# EUROPEAN PATENT SPECIFICATION

(11) **EP 3 849 715 B1**
(45) Date of publication and mention of the grant of the patent: **02.08.2023**
(21) Application number: 19805142.7
(22) Date of filing: 11.09.2019
(51) Int. Cl.: B07C 5/36

(54) **SYSTEM AND APPARATUS FOR PROCESSING AND GRADING FOOD ARTICLES AND RELATED METHOD**
SYSTEM, VORRICHTUNG UND VERFAHREN ZUM BEARBEITEN UND SORTIEREN VON LEBENSMITTELN
SYSTÈME, DISPOSITIF ET PROCÉDÉ DE TRAITEMENT ET DE TRI D'ARTICLES ALIMENTAIRES

(30) Priority: 11.09.2018 US 201862729781 P
(43) Date of publication of application: 21.07.2021
(73) Proprietor: Valka Ehf, 203 Kopavogur (IS)
(72) Inventor: HJALMARSSON, Helgi, 200 Kopavogur (IS); JONSSON, Einar Bjorn, 112 Reykjavik (IS); GUNNARSSON, Hannes, 225 Gardabaer (IS); HERMANNSSON, Ingolfur Harri, 112 Reykjavik (IS); EIRIKSSON, Jon, 200 Kopavogur (IS)
(74) Representative: Vossius & Partner Patentanwälte Rechtsanwälte mbB
(86) International application number: PCT/EP2019/074281
(87) International publication number: WO 2020/053310

(56) References cited:
- FR-A1- 2 595 589
- US-A- 6 015 049

## Description

### TECHNICAL FIELD

Embodiments of the disclosure generally relate to apparatuses and methods for processing and grading food articles, for example, based on at least one characteristic of the food articles, wherein the grading comprises arranging the food articles along a conveyor and/or grading the articles onto one or more receiving areas.

### BACKGROUND

Conventional grading machines include arms and/or trays to grade food articles while the food articles move along a conveyor. Food articles are often graded based on different characteristics, such as weight. Such methods often require several human participants to double check automatic procedures performed by machines, or to perform additional actions not performed by machines.

For example, U.S. Patent 7,258,237 describes a grading technique that includes weighing and portioning an item. In the grading technique, natural foodstuff items with varying weights are subjected to a weighing-in, and are thereafter selectively fed together in a computer controlled manner. According to this reference, a robot device including a grip, operated by a control system, is used for removing items from a delivery station to a receiving area for placement into a particular batch.

Similar methods for poultry packaging are described in "Robotic Packaging of Poultry products, by K. Khodabandehloo, Department of Mechanical Engineering, University of Bristol ISBN 0442316615 (Routledge, 1992)" and "Benefits of Experts robots intelligence vs. Skill, by K. Khodabandehloo, Department of Mechanical Engineering, University of Bristol ISBN 0387537317 (Routledge, 1992)". The previous references disclose a robot used to place poultry portions into trays according to a defined scheme.

FR 2 595 589 A1 discloses a device for separating mushrooms by classifying them into a number of categories, namely short-stemmed, medium-stemmed, and long-stemmed. The device includes a conveyor belt and a motion camera enabling the total length and diameter of each mushroom to be measured. Sorting occurs afterwards on the basis of the ratio of the total length to the diameter. The mushrooms are then trimmed mechanically.

### DISCLOSURE

This summary is provided to introduce a selection of concepts in a simplified form. These concepts are described in further detail in the detailed description of example embodiments of the disclosure below. This Summary is not intended to identify key features or essential features of the claimed subject matter, nor is it intended to be used to limit the scope of the claimed subject matter.

Applicants have discovered that one disadvantage of having a robot arm picking the articles up from a conveyor is that it is difficult to design a good gripper arm to handle (*e.g.,* grasp) delicate food products that typically come in various sizes. If the gripper arm is big enough for the biggest articles, it will require significantly more space between small articles than would otherwise be needed. Furthermore, such gripping robotic arms are not able to grasp articles that are oriented very close to each other on the conveyor. For example, such gripping robotic arms would not be able to grasp fish portions that are cut prior to being graded, but wherein the cut portions are still in the shape of the original fillet, without disturbing or even damaging the surrounding portions.

The present invention relates to a system for grading a food article that has been cut into portions and to a method for grading food articles on a surface as defined by the features of the independent claims, respectively. Variations and further developments are defined by the features of the dependent claims. The surface may be a conveyor having an in-feed end and an out-feed end. Some embodiments comprise cutting a food article *(e.g.,* a fish fillet) into a plurality of portions that are bisected by a line that is parallel to the in-feed end and/or the out-feed end of the conveyor. In embodiments, each portion of a plurality of portions may have an alignment and orientation. Grading a portion of the plurality of portions may include moving at least one portion of the plurality of portions, while at least substantially maintaining the alignment and orientation of each portion of the plurality of portions. In particular examples, moving the at least one portion may remove the portion(s) from a surface whereupon the portions are disposed.

In some embodiments, a method for grading food articles includes cutting a food article into a plurality of portions oriented parallel to each other. Some examples include grading at least one of the plurality of portions that is oriented parallel to at least another of the plurality of portions, wherein the at least another of the plurality of portions is/are not graded. Some examples comprise grading at least one of the plurality of portions that is positioned on the conveyor such that each side of the at least one portion(s) is proximate a side of at least one adjacent portion; e.g., a cut portion is graded from within the middle of the shape of the original food article.

In some embodiments, a food article is cut into the plurality of portions, such that the cut portions are oriented very close to each other on the conveyor (for example, wherein the cut portions are separated by a space that is essentially the width of a blade of the cutting instrument). In some examples, at least one of the very closely oriented portions is graded without damaging and/or changing the orientation and/or alignment of the other portion(s) on the conveyor.

In particular embodiments, a method for grading food articles comprises at least one step selected from the group consisting of: capturing a first x-ray image of a food article; detecting at least a first portion of the food article to be removed, and at least a second portion of the food article to retain using the first x-ray image; cutting the first portion of the food article away from the second portion of the food article; moving the first portion of the food article automatically with a machine; and capturing a second x-ray image of the second portion of the food article. Certain examples include a method comprising all of the foregoing steps.

By using x-ray images in this way, informed detection of the at least a first portion of the food article to be removed is provided. For example, the x-ray image may identify bones in the food article and the at least a first portion may be detected based on the locations of the bones in the food article. Accordingly, a more accurate system and method is provided.

In particular embodiments, a method for grading food articles comprises at least one step selected from the group consisting of: cutting a food article into a plurality of portions oriented parallel to each other, wherein each portion has an alignment and orientation; capturing an x-ray image of the plurality of portions oriented parallel to each other; and moving less than all of the plurality of portions automatically with a machine. Some examples comprise detecting which portions of the plurality of portions contain an undesirable component of the portion (for example, with a first x-ray machine); and moving only portions of the plurality of portions containing an undesirable component. In particular examples, the undesirable component is selected from the group consisting of bones *(i.e.,* a bone or bone fragment), cartilage, fat, defects in flesh, tough tissues, skin, blood, and organs. In certain examples, the undesirable component is a bone. In certain embodiments, at least one detected portion containing an undesirable component is moved automatically, and any remaining portion(s) comprising a further undesirable component are detected (for example, in a second x-ray machine).

Some embodiments include a grading device *(i.e.,* a robot), which grading device may have any number of degrees of freedom. In particular embodiments, the grading device may comprise a horizontally movable support member; a vertically movable support member slidably coupled to the horizontally movable support member; a first actuator attached to the horizontally movable support member; a second actuator attached to the vertically movable support member; and a means for moving at least one portion of a food article attached to the vertically movable support member (e.g., a needle array, and a gripper). In some examples, the means for moving at least one portion of a food article may be utilized to move a plurality of portions at substantially the same time *(i.e.,* in one movement of the grading device).

Some embodiments include an automated food processing system that contains, in sequential order on a conveyor, a first x-ray machine, at least one cutting machine, and a second x-ray machine. In some embodiments, the cutting machine(s) is adapted to cut out a portion containing a bone or bone fragment from a food article, leaving one or more portions of the food article containing flesh. In some examples, the automated food processing system comprises computer programming to utilize information from the second x-ray machine to locate a bone or bone fragment in a food article portion, and a computer adapted to adjust the operation of the cutting machine(s) according to the location of a bone or bone fragment in a portion of a food article, so as to maximize the size of the one or more portions of the food article containing flesh that remain(s) after cutting. In particular examples, the automated food processing system comprises at least one grading device (*e.g*., one, two, three, four, five, or more grading devices) that is adapted to move a portion of the food article containing bones after cutting, as determined by the second x-ray machine; for example, to reposition the portion on the conveyor before the cutting machine(s), or to remove the portion from the conveyor. The grading device(s) may be positioned anywhere in the automated food processing system; for example, following an x-ray machine or conveyor.

The use of x-rays to inform the cutting pattern adopted by the cutting machine results in a more accurate, and thus less wasteful, approach for removing undesired components of a food article. This is because the cutting can be based on highly accurate x-rays of the food article showing, for example, areas of bone or bone fragment. Further, advantages of the second x-ray machine include (a) use of the second x-ray machine means that a feedback loop is provided such that the cutting pattern can be adjusted if the second x-ray machine shows that the portion to be removed can be smaller (thus leaving more useful flesh), and (b) the fact that results from the second x-ray machine can be used to inform a subsequent grading device or devices.

In some embodiments, an automated food processing system comprises computer programming to utilize information from the first x-ray machine to locate a bone or bone fragment in a food article portion. In particular examples, the automated food processing system comprises at least one grading device that is adapted to move a portion of the food article containing bones after cutting, as determined by the first x-ray machine; for example, to reposition the portion on the conveyor before the cutting machine(s), or to remove the portion from the conveyor.

In some embodiments, the grading robot may include a first mounting member mounted to a side of a grading conveyor and a second mounting member mounted to a side of the grading conveyor (for example, a side other than that to which the first mounting member is mounted). The grading robot may include a first guide member horizontally mounted to both the first mounting member and the second mounting member, and a horizontally movable support member slidably coupled to the first guide member. The grading robot may include a second guide member mounted to the horizontally movable support member, and a vertically movable support member slidably coupled to the second guide member. The grading robot may in some examples comprise at least one additional actuator, so as to provide at least one additional degree of freedom. Furthermore, the grading robot may include a first actuator (*e.g*., air cylinder, motor, linear motor, traditional motor, and solenoid) attached at one end to the horizontally movable support member. The first actuator may be attached at another end to the second mounting member. The grading robot may include a second actuator attached at one end to the second guide member. The second actuator may be attached at another end to the vertically movable support member. In some examples, a grading robot may include at least one needle or gripper attached to the vertically movable support member.

Advantageously, the more degrees of freedom that the grading robot has, the more adaptable it is to changing requirements. For example, the more adaptable it is to the changing shapes and sizes of food articles and portions within such food articles.

Some embodiments include at least one realigning apparatus for aligning food article. In particular embodiments, a realigning apparatus for aligning a food article or portion of a food article may comprise a first mini-conveyor; a second mini-conveyor disposed proximate the first mini-conveyor; a first actuator attached to the first mini-conveyor; and a second actuator attached to the second mini-conveyor, wherein the first mini-conveyor is mounted with a hinge such that the first mini-conveyor is separable from the second mini-conveyor. In some embodiments, a realigning apparatus may include a first mini-conveyor disposed above a processing conveyor, and a second mini-conveyor disposed above the processing conveyor proximate the first mini conveyor. Both the first mini-conveyor and second mini-conveyor may be tilted at an angle, such that the first mini-conveyor and second mini-conveyor form a V-shape.

The provision of such a realigning apparatus means that remaining portions in a food article may be realigned after, for example, the removal of a portion using the grading machine. Realignment of remaining portions may be necessary for the efficient and accurate completion of subsequent processes such as packing and freezing.

Some embodiments include a food processing apparatus. In particular embodiments, a food processing apparatus comprises at least one conveyor; at least one grading device (*e.g*, a robot); and at least one imaging system, at least one cutting machine, and/or at least one realigning apparatus. In particular embodiments, the food processing apparatus comprises at least one of: x-ray machine(s), manual quality check station(s), automated quality check station(s), and realigning apparatus. Certain embodiments include a food processing apparatus comprising at least one conveyor; at least one x-ray machine; a first imaging system; at least one cutting machine; at least one quality check station; at least one additional imaging system; at least one grading device; at least one realigning apparatus; and at least one computer programmed to utilize information from the additional imaging system(s) to determine, for example, the location of a bone or bone fragment in a portion of a food article, the location of fat in a portion of a food article, the color of a portion of a food article, the location of a gap in a portion of a food article, the location of a visual defect in a portion of a food article, and the location of parasites in a portion of a food article, wherein the computer is programmed to utilize information about the movement of the at least one conveyor and any determined feature to adjust the position of the means for moving at least one portion of a food article of the grading device.

The additional imaging system can therefore be used to accurately inform the removal of undesirable portions of the food article by the grading device. This helps to ensure both that undesirable portions are removed, and desirable portions not removed; *i.e.,* left ready for subsequent processing.

According to a first aspect, there is provided a system for grading a food article that has been cut into portions, the system comprising: a first conveyor; at least one spray-removal robot; a first receiving conveyor and a second receiving conveyor, defining a space therebetween; at least one spray-removal robot; and a third receiving conveyor, wherein the spray-removal robot comprises at least one valve with a nozzle, and wherein the valve ejects fluid through the nozzle to impact a food article being moved on the first conveyor toward the third receiving conveyor, such that at least one portion of the food article is moved by the impact through the space between the first receiving conveyor and the second receiving conveyor, and such that at least one portion of the food article is moved by the motion of the first receiving conveyor and the second receiving conveyor to the third receiving conveyor.

The spray-removal robot may comprise a plurality of valves with nozzles. Each valve may have a respective nozzle. Each of the plurality of valves with nozzles may be independently controllable. The spray-removal robot may comprise a plurality of nozzles, and optionally a plurality of valves. Each nozzle may have a respective valve. Each valve and/or nozzle may be independently controllable. Advantageously, by providing a plurality of nozzles, each with a respective valve, an improved spray-removal robot is provided. This is because greater control of the ejection of fluid from the robot is provided because fluid ejection may be controlled on an individual nozzle level. In turn, this allows for greater control over the movement of the at least one portion of the food article. For example, if the at least one portion is very narrow, fluid only need be ejected from one nozzle. Usefully, this avoids an overly large fluid stream being ejected that, if too large, would impact upon and disturb other portions of the food article. Conversely, if the at least one portion is very wide, fluid may be ejected from multiple ones of the nozzles such that the at least one portion is impacted over a wide surface area to easily move the portion through the space between the first and second receiving conveyors.

The spray-removal robot may comprise a plurality of nozzles. The nozzles may be arranged in a cluster; each nozzle may be adjacent or in close proximity to at least one other nozzle. The net shape of the cluster may be triangular and/or substantially match the net shape of the at least one portion. There may be a right nozzle, a left nozzle and a centre nozzle. Fluid may be ejected from each nozzle alone, or in combination with any others of the nozzles. The net shape of the fluid stream ejected from the nozzles may approximately match the net shape of the at least one portion in at least one dimension. The net shape of the fluid stream ejected from the nozzles may approximately match the net shape of the at least one portion. These features provide an accurate and highly controllable spray-removal robot. This is because the size and shape of the stream of fluid may be accurately controlled. For example, if a wide stream of fluid is needed (to impact upon a wide portion, for example), both the right and left nozzles may be used, say; whereas if a narrow but long stream of fluid is needed (to impact upon a narrow portion, for example), the right and centre nozzle may instead be used. Accordingly, the fluid ejected from the robot can be accurately controlled to substantially match the shape and size of the portion to be removed and so avoid impacting upon, and thus distributing or damaging, other portions of the food article.

The system may comprise a plurality of the spray-removal robots.

The first receiving conveyor and the second receiving conveyor may be movable in a direction substantially perpendicular to the movement of the first conveyor. The movement of the first receiving conveyor and the movement of the second receiving conveyor may be independently controllable. The movement of the first receiving conveyor and the movement of the second receiving conveyor may be controlled by a computer that controls the spray-removal robot. The respective locations and/or positions of the first and second receiving conveyors may be independently controllable such that the size and/or position of the space between the first and second receiving conveyors may be adjusted. The size and/or position of the space between the first and second receiving conveyors may be adjusted based on the size and/or position of the at least one portion. The size and/or position of the space between the first and second receiving conveyors may be adjusted before, or substantially at the same time as, the food article moves from the first conveyor to the first and second receiving conveyors. In use, when the food article has moved from the first and second receiving conveyors to the third receiving conveyor, the size and/or position of the space between the first and second receiving conveyors may be adjusted based on the next food article to be received on the first and second receiving conveyors. Advantageously, the provision of moveable first and second receiving conveyors means that an improved spray-removal robot is provided. This is because the space between the first and second receiving conveyors can be adapted based on the size and/or position of the at least one portion such that the at least one portion may easily fall through the space, whilst the rest of the food article is safely supported on the conveyors for subsequent processing. This prevents both the at least one portion from getting caught on the conveyors and not successfully moving through the space, and the other portions from inadvertently falling through the space.

The at least one portion may have a width in the direction substantially perpendicular to the movement of the first conveyor. The at least one portion may have a length in the direction substantially parallel to the movement of the first conveyor. The at least one portion may be longer than it is wide. The width of the space may be substantially the same as the width of the at least one portion. The width of the space may be larger than the width of the at least one portion. The first and second receiving conveyors may be controlled to substantially follow the path of the edge of the portion to be removed. That is, to substantially follow the path of the width of the portion to be removed. In other words, the positions and locations of the first and second receiving conveyors may be controlled such that the space between them is substantially equal to or follows the width of the at least one portion as the at least one portion is received on the first and second receiving conveyors. Advantageously, by dynamically varying the space between the conveyors to follow the width of the at least one portion, the at least one portion is encouraged to fall through the space because it is not supported by the first and second receiving conveyors; however, the other portions of the article are prevented from falling through the space, because they are supported by the first and second receiving conveyors.

The first receiving conveyor and the second receiving conveyor may be rollers. The rollers may rotate about an axis or respective axes. The axis or axes of the rollers may be perpendicular to the direction substantially parallel to the movement of the first conveyor. Accordingly, each roller may be movable in a direction parallel to its axis. Advantageously, the provision of rollers results in a spray-removal robot of reduced overall size. This is because the overall footprint of the rollers is much smaller than that of flat conveyors, for example. Further, rollers contribute to maintaining the alignment of the food article as the rollers help to avoid portions of the food article from bunching together as the portions move between conveyors. For example, rollers help to stretch out (in the direction of movement of the conveyors) the portions of the food article. Accordingly, the provision of rollers results in an improved spray-removal robot.

The first and second receiving conveyors may be vertically disposed between the first conveyor and the third receiving conveyor. That is, the first conveyor may be substantially arranged at a first height, the first and second receiving conveyors may be substantially arranged at a second height, and the third receiving conveyor may be substantially arranged at a third height, and wherein: the first height is greater than the second height, and the second height is greater than the third height. Advantageously, the arrangement of the first and second receiving conveyors between the first conveyor and the third receiving conveyor helps the at least one portion to move through the space between the conveyors. This is because, due to the height difference, the at least one portion impacts upon the first and second receiving conveyors as it departs from the first conveyor and this impact can help dislodge the at least one portion and help it to move through the space. Furthermore, the height difference also helps to maintain the alignment of the portions and prevent them from bunching up or getting caught between conveyors as gravity naturally helps the food article move from one conveyor to the next. And finally, the arrangement of the conveyors at different heights is an energy efficient solution since, as discussed, gravity helps to move the food articles from one conveyor to the next, thus mitigating the need for additional electrically powered aids, for example.

The system may further comprise a laser sensor.

According to a second aspect, there is provided a food processing apparatus comprising: at least one conveyor; a cutting machine; and the system according to the first aspect for grading a food article that has been cut into portions.

The food processing apparatus may further comprise: at least one element selected from the group consisting of at least one imaging system; at least one realigning apparatus; at least one x-ray machine; at least one manual quality check station; at least one automated quality check station; at least one additional grading device; and at least one realigning apparatus.

The additional grading device may be a needle grading device, a gripper grading device, a two degrees of freedom grading device, a three degrees of freedom grading device, or a four degrees of freedom grading device.

The food processing apparatus may further comprise at least one computer programmed to operate the spray-removal robot according to one or more inputs selected from the group consisting of an x-ray image, 3D image, a signal from a cutting robot, the movement of the grading conveyor, and the movement of any other conveyor(s) preceding the spray-removal robot.

According to a third aspect, there is provided a method for grading a food article that has been cut into portions, the method comprising utilizing the system according to the first aspect for grading a food article to remove at least one of the portions of the food article through the space defined between the first receiving conveyor and the second receiving conveyor, while transferring the remaining portions of the food article to the third receiving conveyor.

The action of the spray-removal robot may be controlled by a computer according to a determination of a location of the portion to be removed, wherein the computer determines the location of the portion to be removed from one or more inputs selected from the group consisting of an x-ray image, 3D image, a signal from a cutting robot, the movement of the grading conveyor, and the movement of any other conveyor(s) preceding the spray-removal robot.

Each of the portions may have an alignment and orientation with respect to the surface of the first conveyor, and may be positioned parallel to each other on the surface of the first conveyor, wherein none of the portions that are not removed and are positioned parallel to each other on the surface of the first conveyor may be substantially moved during the removal of the portion through the space defined between the first receiving conveyor and the second receiving conveyor, and wherein the alignments and orientations of the portions that are not removed and are positioned parallel to each other on the surface of the first conveyor may be substantially maintained after the removal of the portion through the space defined between the first receiving conveyor and the second receiving conveyor, and the transfer of the remaining portions that are not removed to the third receiving conveyor.

The portion that is removed may be closely adjoined on opposing sides in an orientation substantially perpendicular to the parallel orientation of the plurality of portions by other portions.

The method may further comprise selecting the portion to be removed based on at least one criteria selected from the group consisting of size, weight, quality, and type. The criteria may be the type of comprising bone. That is, the criteria may be type and the type may be comprising bone.

Features of the disclosed embodiments or aspects may be combined to form further embodiments or aspects. For example, a feature or features of an embodiment or aspect may be combined with a feature or features of any other embodiment(s) or aspect(s). Optional features of each aspect or embodiment may be optional features of each other aspect or embodiment.

### BRIEF DESCRIPTION OF THE DRAWINGS

The present disclosure may be understood more fully by reference to the following Detailed Description of several embodiments, which are illustrated in the appended figures, in which:

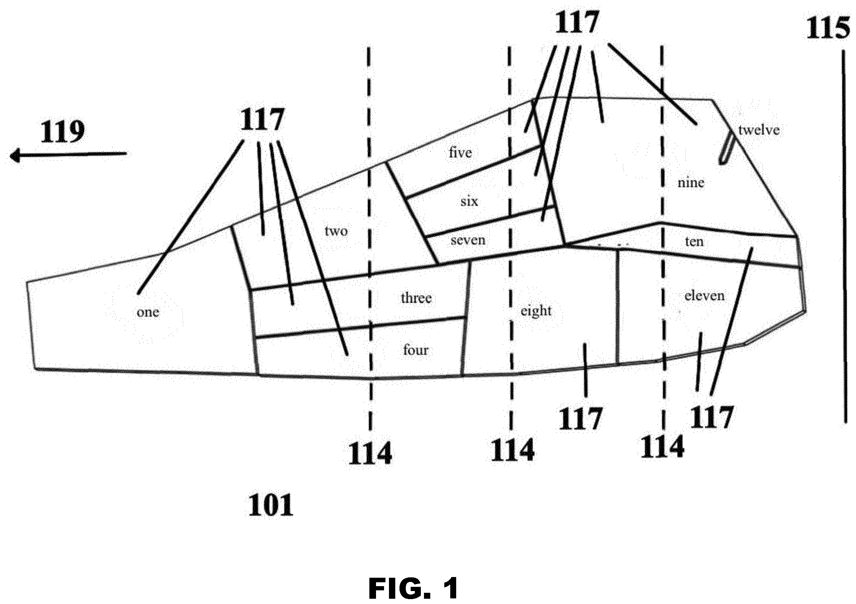

**FIG. 1** includes a top view of an example of a food article **101** (*i.e.,* a fish fillet) cut into portions **117**, wherein included in the shape of the original food article **101** is a first plurality of portions **117** ("two," "three," and "four"), a second plurality of portions ("five," "six," "seven," and "eight"), and a third plurality of portions ("nine," "ten," and "eleven"), each of which is bisected by a line **114** parallel to out-feed end **115** of the conveyor upon which the article **101** is disposed. Referring to **FIG. 1**, portions **117** "three," "six," and "seven" are portions **117** that are positioned such that each side is proximate a side of at least one adjacent portion **117**. Referring again to **FIG. 1**, portions **117** "one" and "eight" are in series with portion **117** "three," and portions **117** "two" and "four" are parallel to portion **117** "three."

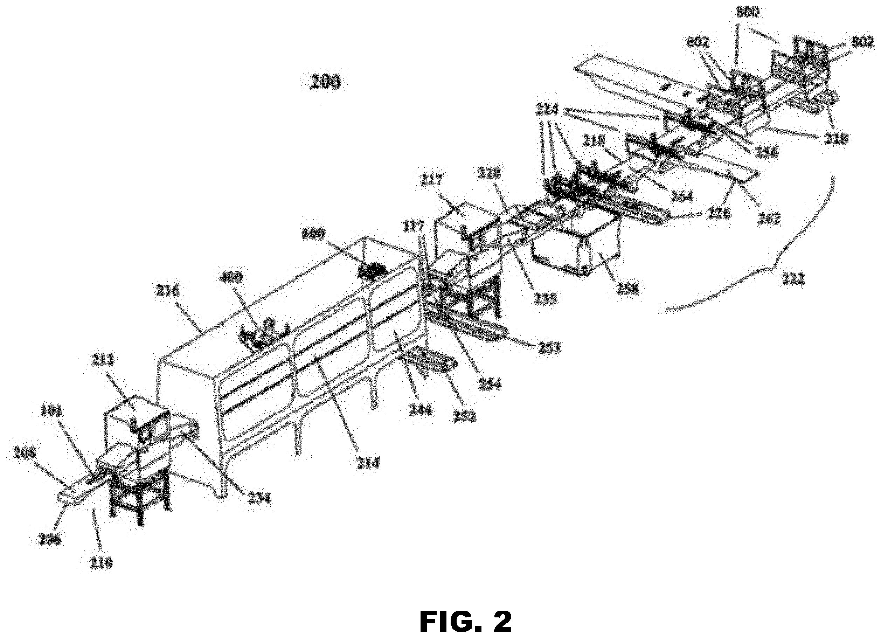

**FIG. 2** includes a perspective view of an example of a food processing apparatus.

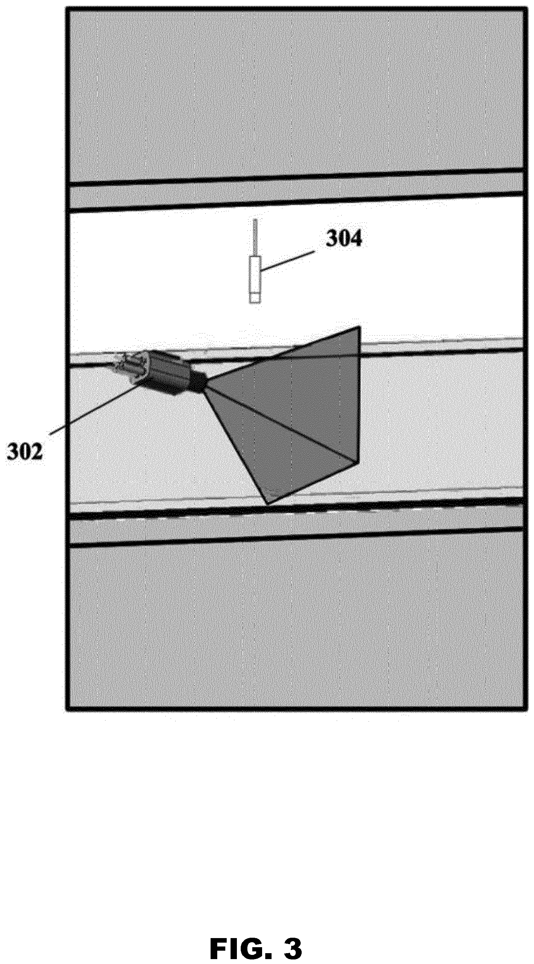

**FIG. 3** includes a perspective view of an example of a 3D camera of an imaging system of a food processing apparatus.

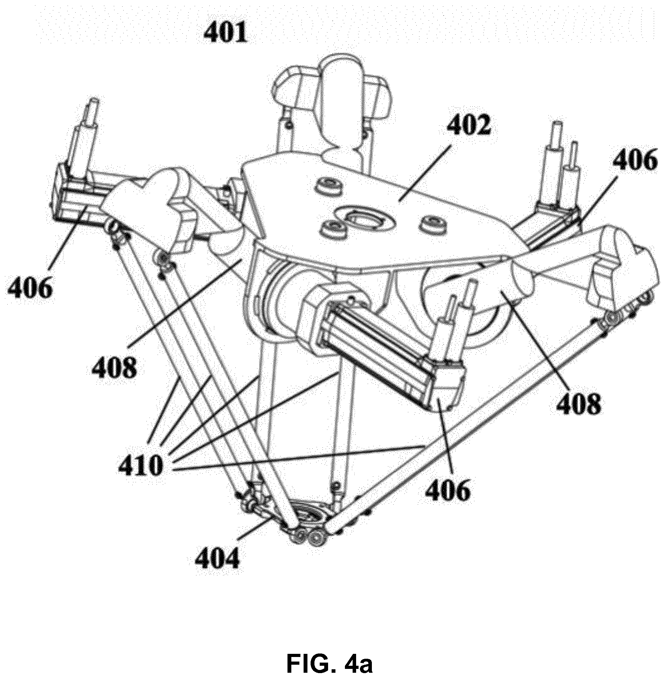

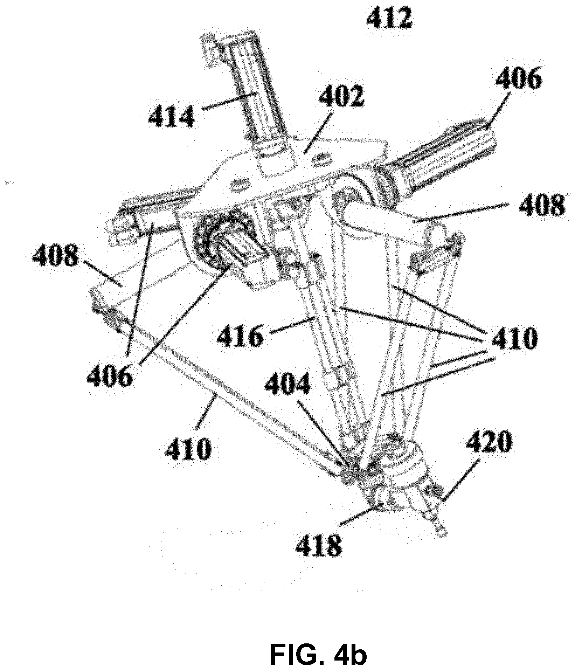

**FIG. 4****(a-b)** includes perspective views of illustrative cutting machines of a food processing apparatus. **FIG. 4a** includes a perspective view of an example of a "delta" or "spider" cutting robot *(i.e.,* a three degrees of freedom cutting robot). **FIG. 4b** includes a perspective view of an example of a four degrees of freedom cutting robot.

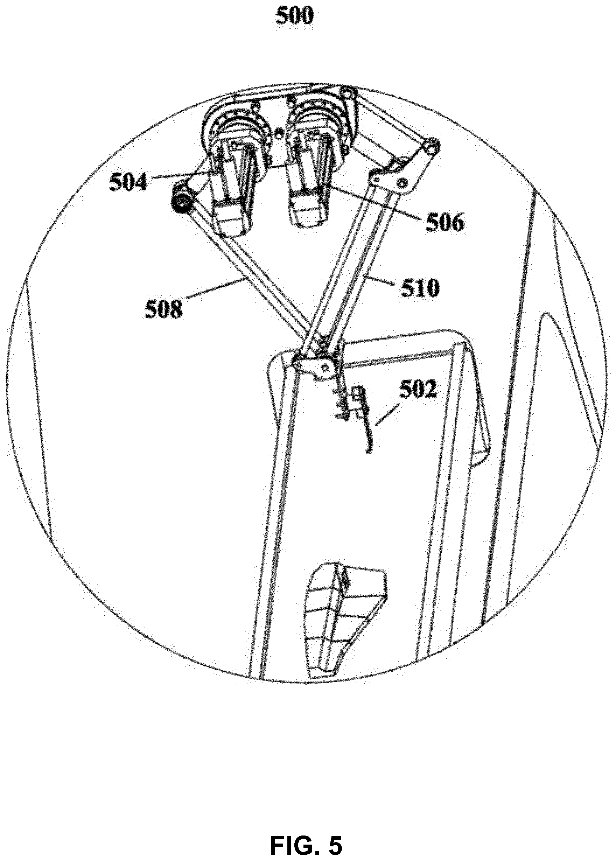

**FIG. 5** includes a perspective view of an example of a robotic arm of a food processing apparatus.

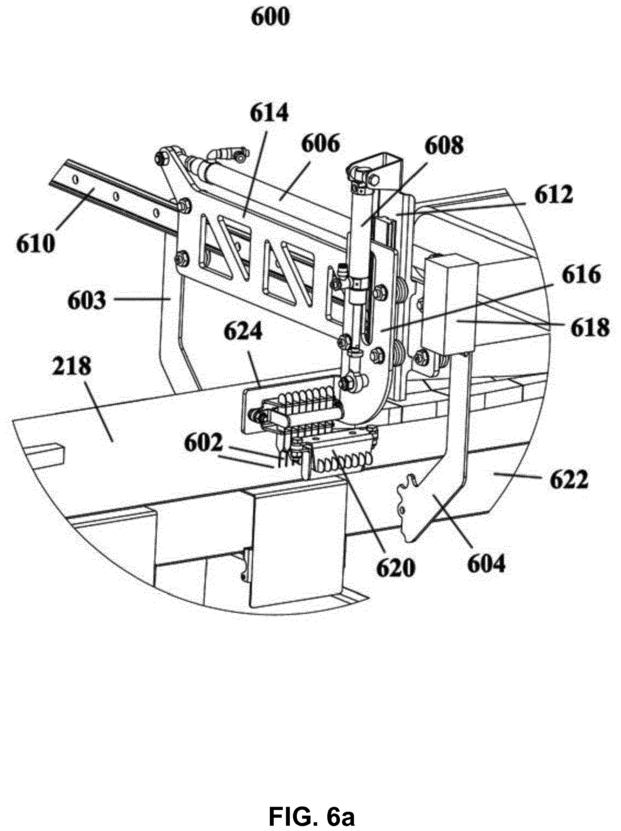

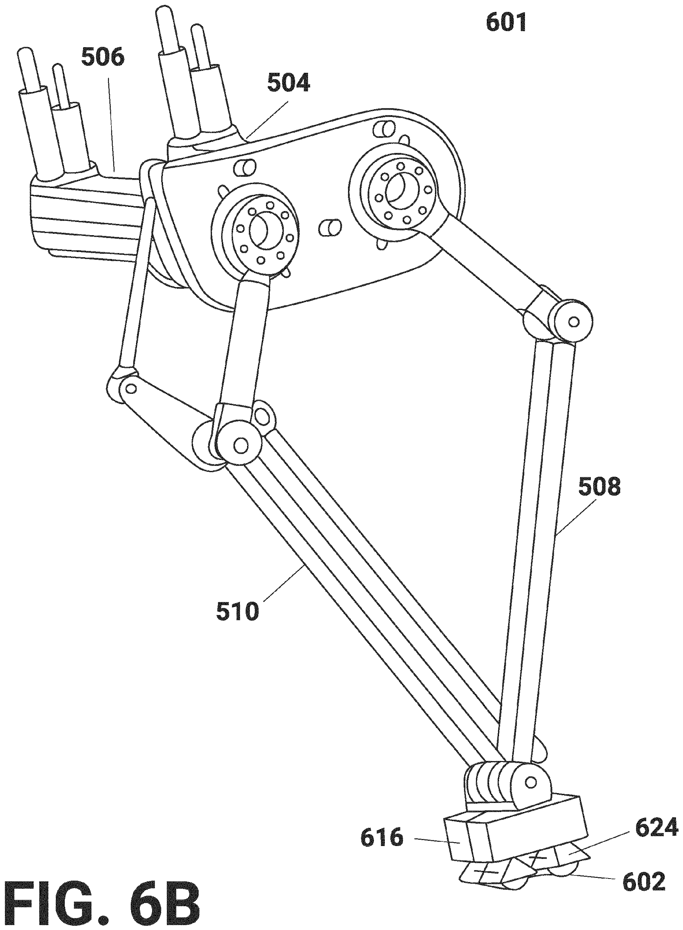

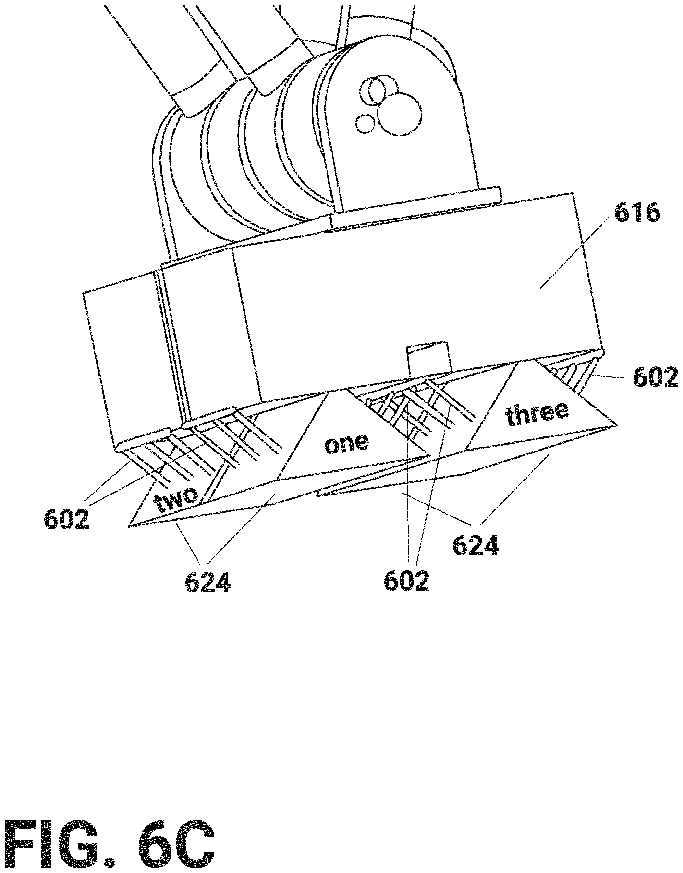

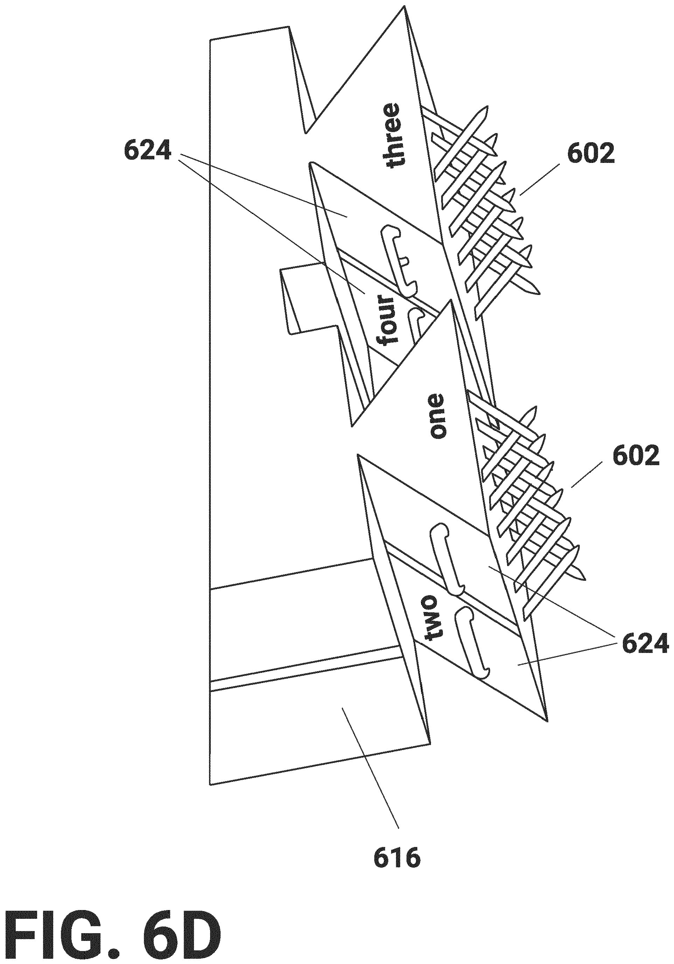

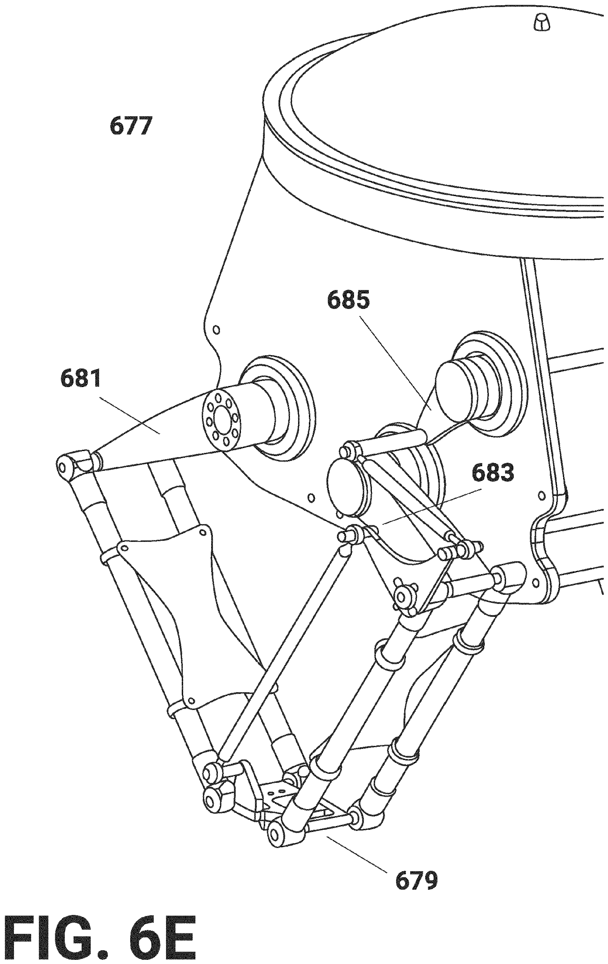

**FIG. 6a** includes a perspective view of an example of a needle grading robot of a food processing apparatus. **FIG. 6b** includes a perspective view of a further example of a needle grading robot of a food processing apparatus. **FIG. 6****(c-d)** include perspective views of a representative means for moving at least one portion of a food article attached to a vertically movable support member. In this particular example, the means for moving at least one portion of a food article attached to a vertically movable support member is an example of a needle array attached to a lowermost portion of a vertically movable support member. In **FIG. 6c****,** the needles of the array are positioned proximate the vertically movable support member. In **FIG. 6d**, the needles of the array are positioned distal the vertically movable support member.
FIG. 6e includes a perspective view of an example of a three degrees of freedom robot **677** that may be utilized in some embodiments, for example, as a spray-removal robot **651**. In particular embodiments, a three degrees of freedom robot **677** comprises a first primary axis **681** and a second primary axis **683** that can control the location of a parallel plate **679** in the Y and Z directions, and a third axis **685** that can tilt the parallel plate **679** in a plane that is perpendicular to the direction of the conveyor.

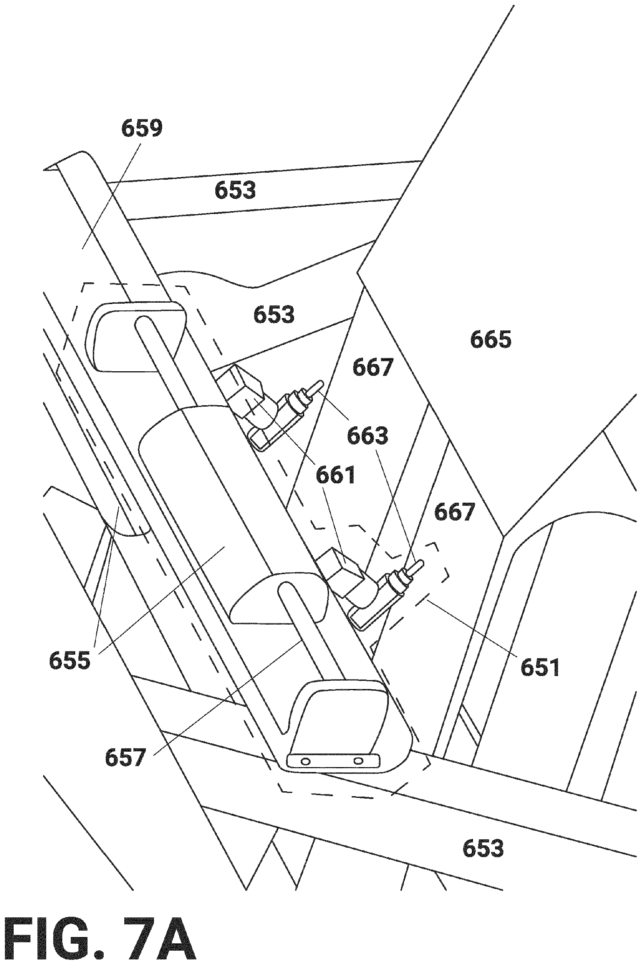

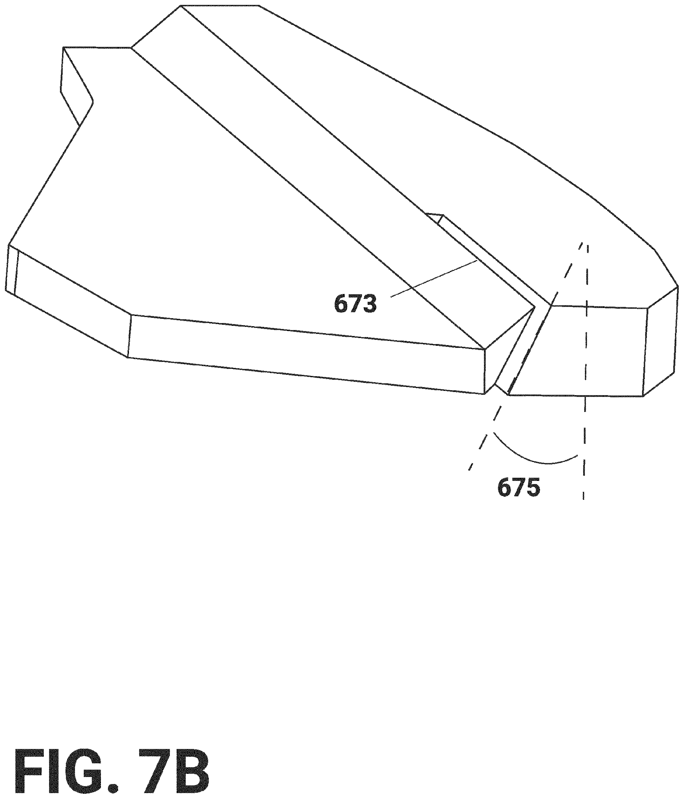

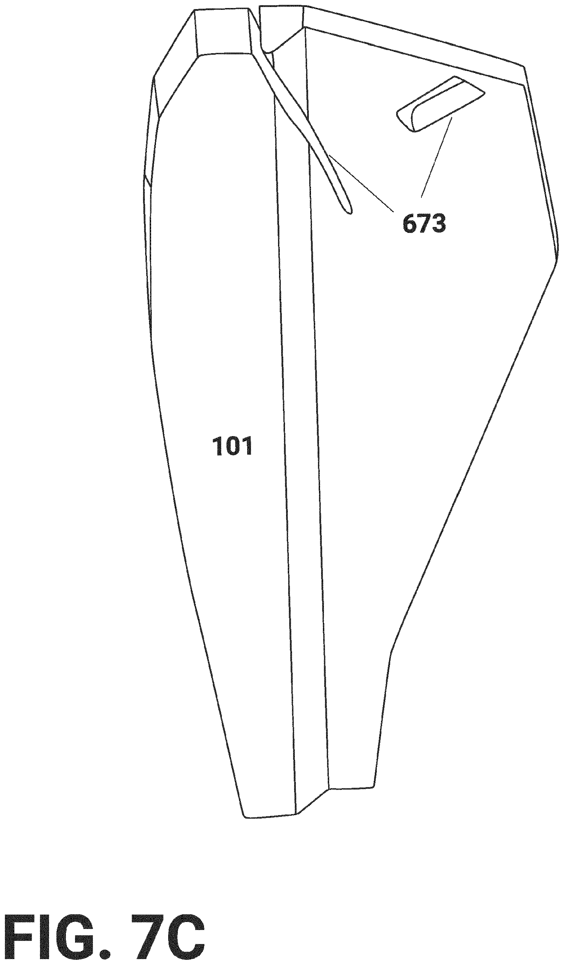

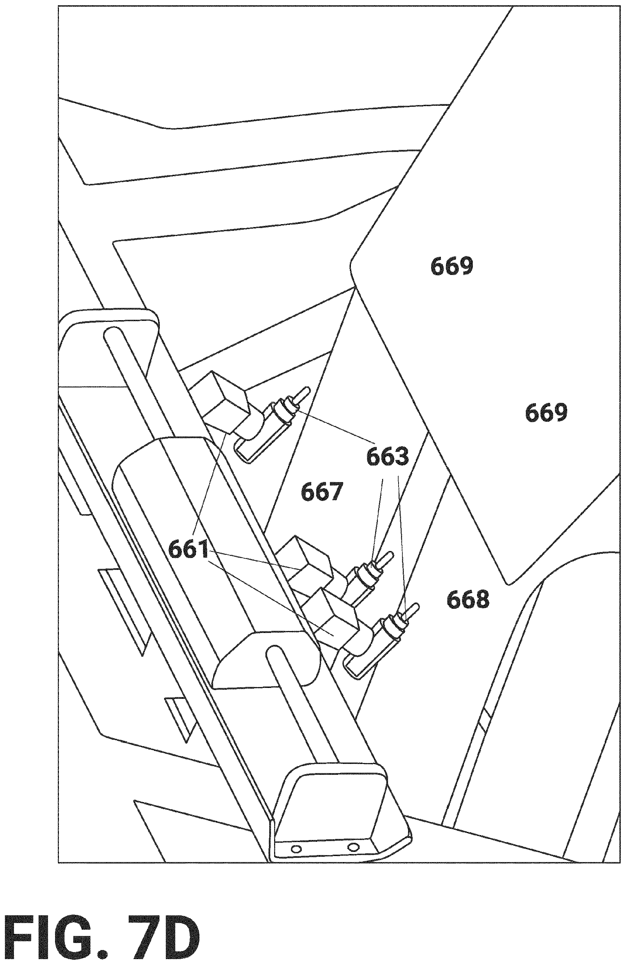

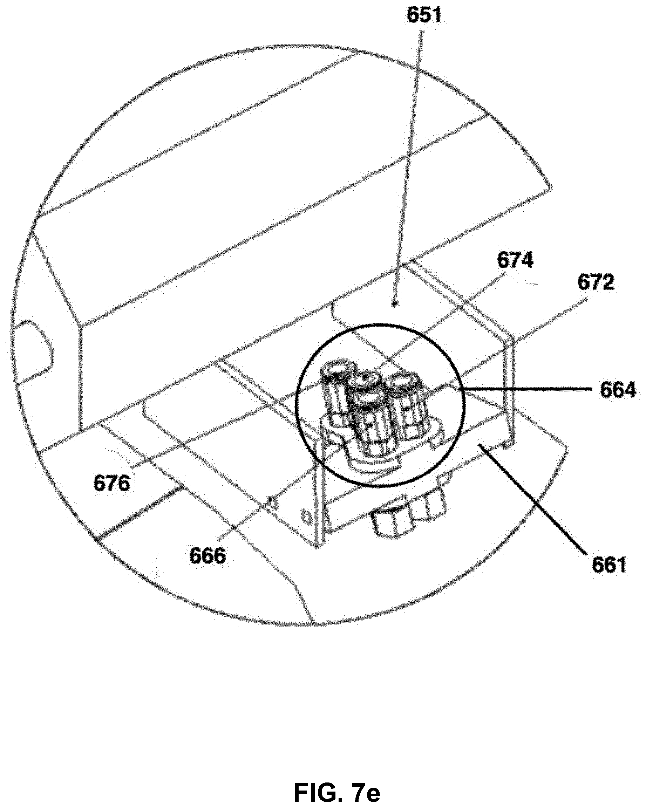

**FIGs. 7-8** illustrate means for the removal of a portion **117** that has already been cut from a fillet **101** by the operation of a spray-removal robot **651** in a spray-removal grading section of a food processing apparatus **200**.
**FIG. 7a** includes a perspective view of a spray-removal grading section of a food processing apparatus **200**. In some embodiments, a spray-removal robot **651** in the spray-removal section uses a stream of fluid (*e.g*., water) to remove a cut food portion **117** from a conveyor system including a first conveyor **665**, for example, at an angle, as shown in **FIG. 7b**. Referring to **FIG. 7c**, in some modes of operation, the ejection of fluid from the nozzle **663** can be controlled, such that a portion **117** defined by a partial cut **673** (*i.e*., starting and/or ending within the fillet **101**) is made, for example, to leave the fillet **101** intact around the volume of the cut.
**FIG. 7d** includes a perspective view of a spray-removal section of a food processing apparatus **200** that comprises a plurality of spray-removal robots **651** that forces at least one food portion **117** on the same area **669** of a first conveyor **665**, into the space between the first conveyor **665** and a receiving conveyor **667**. **FIG. 7e** includes a perspective view of a spray-removal robot **651** that comprises a spray array **664** containing a plurality of spray valve/nozzle elements (*e.g*., a left spray nozzle **666**, a right spray nozzle **672**, a center spray nozzle **674**, and/or a second center spray nozzle **676**).

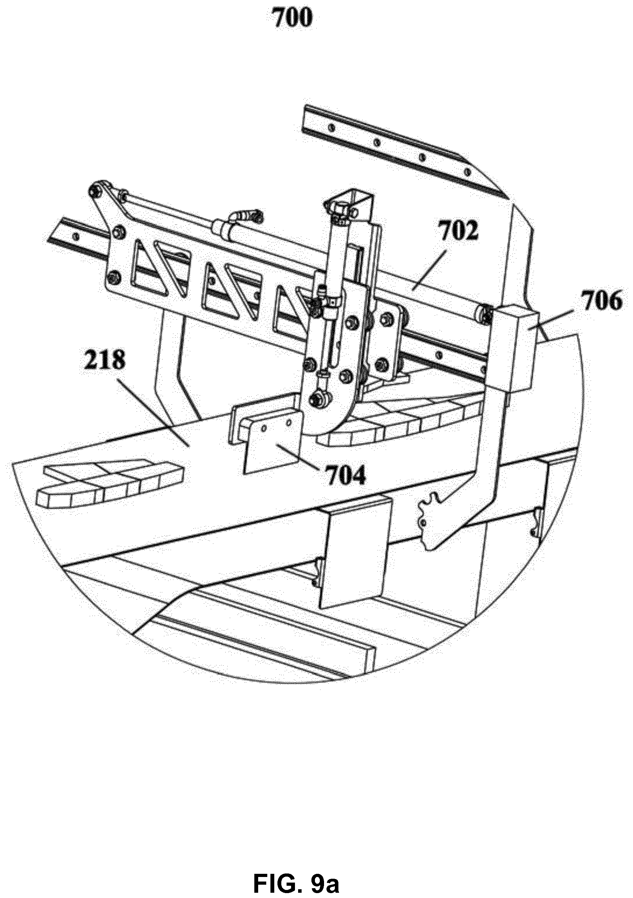

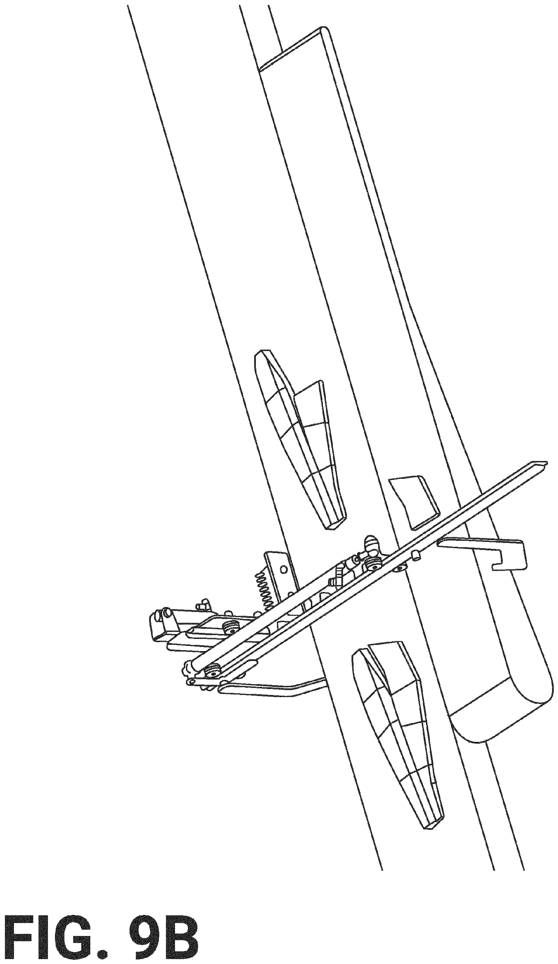

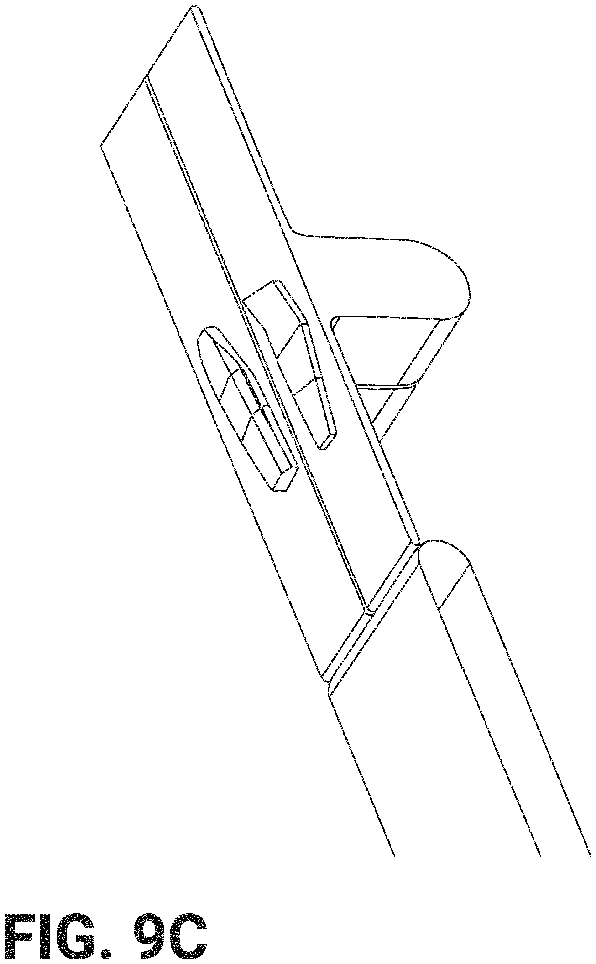

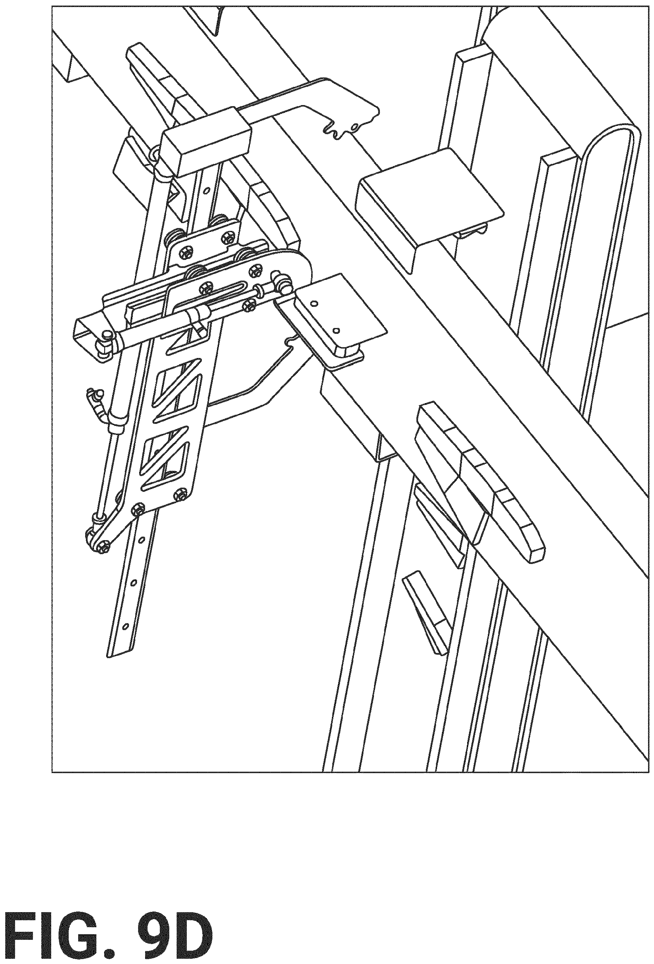

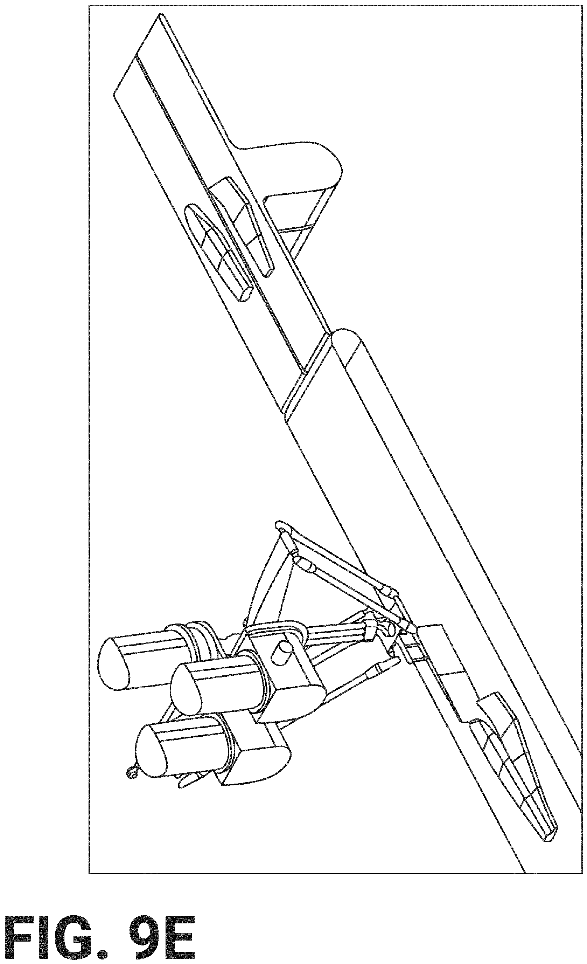

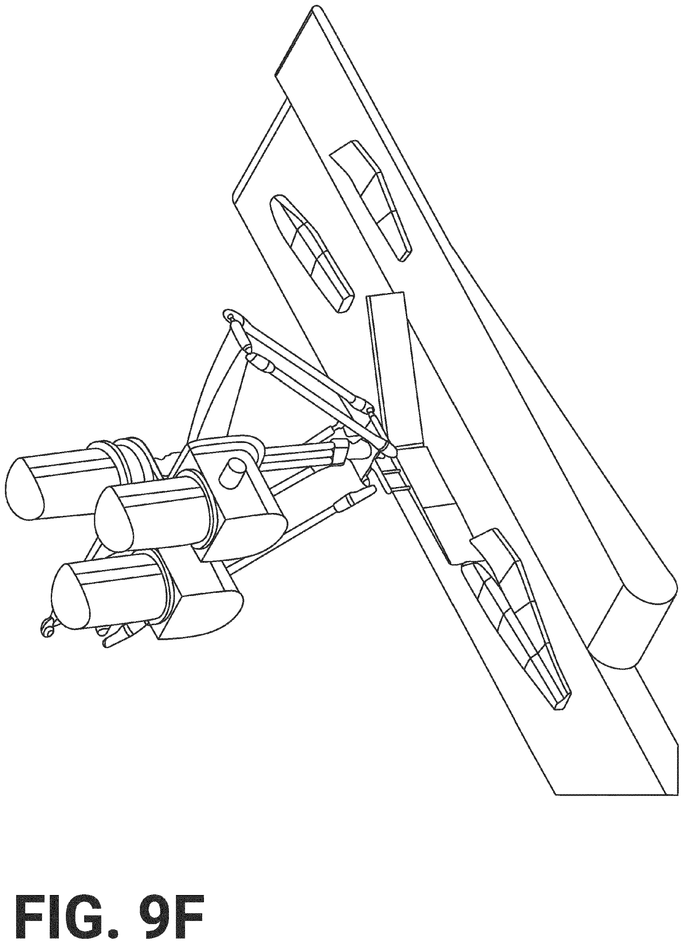

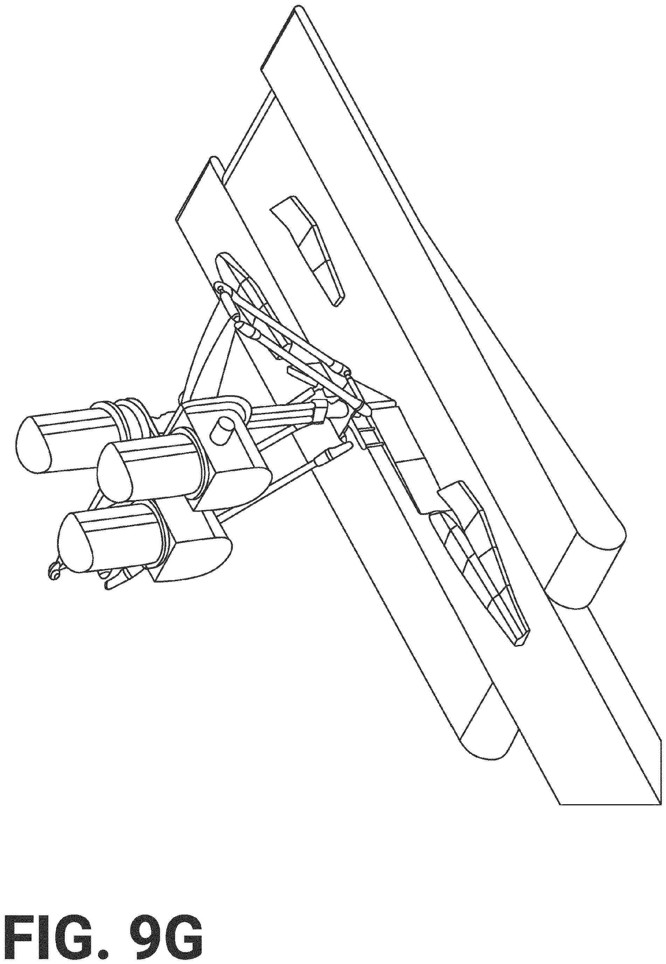

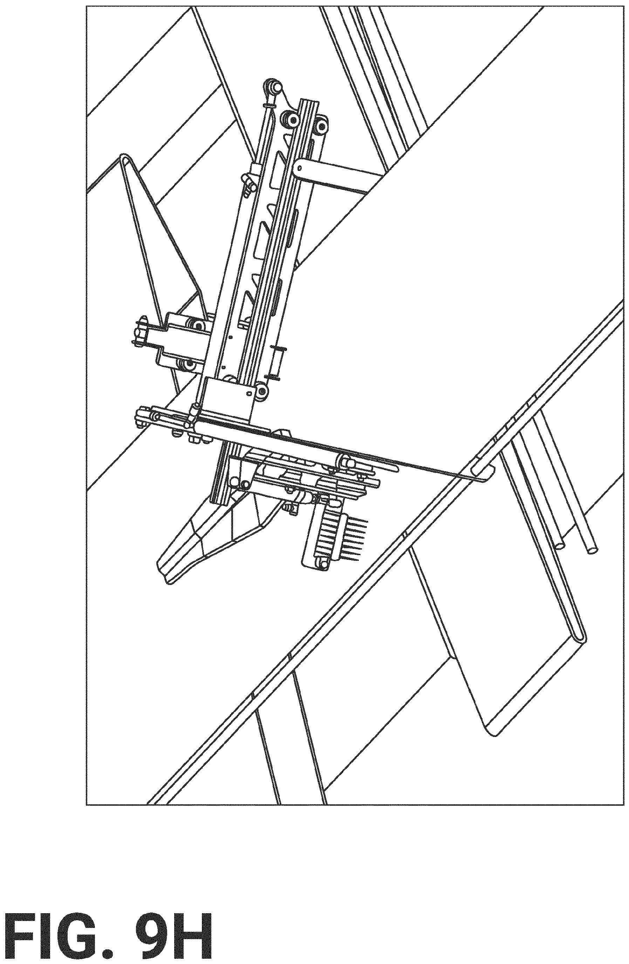

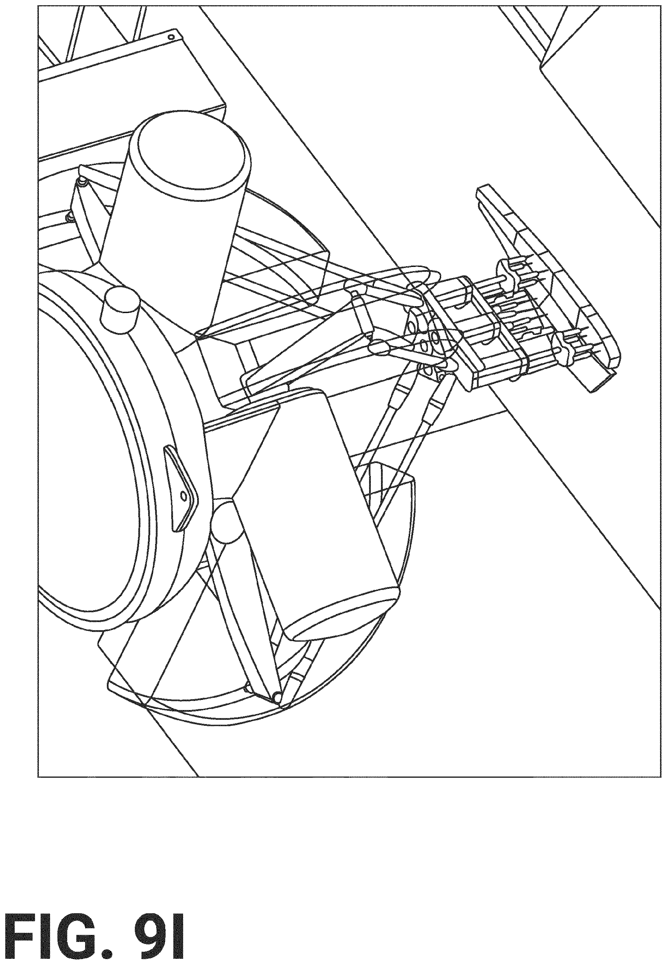

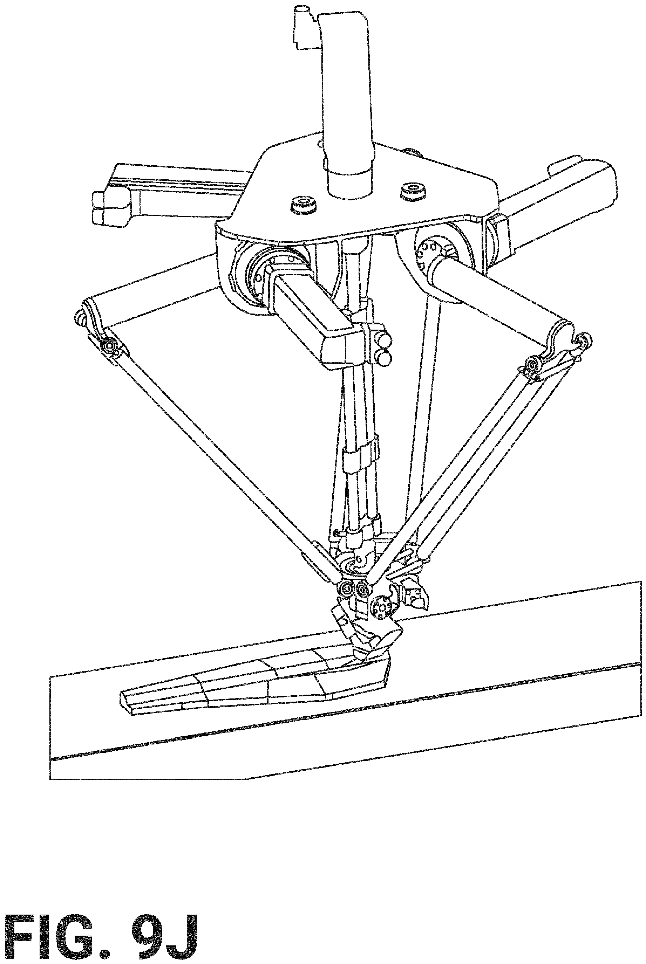

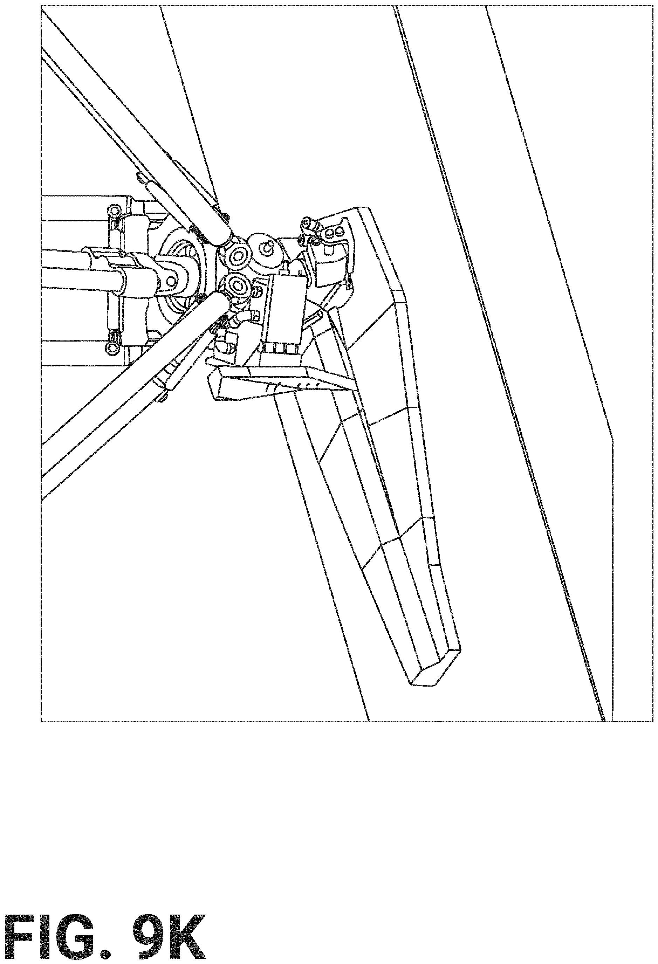

**FIG. 9a** includes a perspective view of a two degrees of freedom grading robot of a food processing apparatus. **FIG. 9b** includes a view of the two degrees of freedom grading robot disposed to transfer at least one portion of the food article from a first conveyor to a second conveyor. In this example, the second conveyor is oriented in parallel with respect to the first conveyor. However, the second conveyor may alternatively be oriented perpendicularly, or at any other angle, with respect to the first conveyor. Furthermore, any grading robot (*e.g*., one, two, three, four, or more degrees of freedom grading robots described herein) may be utilized to transfer a portion of the food article from a first to a second conveyor. Referring again to **FIG. 9b**, in this illustration only one portion is transferred from a first to a second conveyor. However, any number of portions may be transferred, depending on the particular grading robot utilized. Conveyor belts may be positioned to keep the transfer between the belts as smooth as possible. Furthermore, a middle plate can potentially be included, and the second conveyor may in some examples be positioned a little bit lower than the first conveyor that originally comprises the portion(s) on its surface. **FIG. 9c** includes a perspective view of a further represented conveyor system. In this example, a second conveyor and a third conveyor are oriented to follow the first conveyor in its direction of movement. In this example, a grading robot may separate one or more portions of a food article from the remaining portions on the surface of the first conveyor, such that the movement of the conveyor guides the separated portions onto the second conveyor, while guiding the remaining portions onto the third conveyor. **FIGs. 9****(d-f)** include perspective views of a grading robot with a plate transferring portions of a food article from a first to a second conveyor. In **FIG. 9(d)**, the grading robot transfers portions from the first conveyor to a second conveyor oriented perpendicularly with respect to the first conveyor. In **FIG. 9(e)**, the grading robot transfers portions from the first conveyor to a second and a third conveyor that are oriented to follow the first conveyor in its direction of movement. **FIGs. 9****(f-g)** include perspective views of a two degrees of freedom grading robot comprising a first, splitting plate in the direction perpendicular to the conveyor, and a second plate at the end of the first, splitting plate that can be rotated. **FIG. 9(f)** shows an example of rotation of the second plant being utilized to move portions of the food article from a first to a second conveyor. In this example, rotation of the plate moves a plurality of portions to a second conveyor oriented in parallel and on the right side with respect to the first conveyor. **FIG. 9(g)** shows an example of rotation of the second plate being utilized to move portions of the food article from a first to one of two further conveyors. In this example, rotation of the plate in one direction moves a plurality of portions to a second conveyor oriented in parallel and on the left side with respect to the first conveyor. In this example, rotation of the plate in the other direction would move the plurality of portions to a third conveyor oriented in parallel and on the right side with respect to the first conveyor. In some examples, a grading robot comprising a plate may have a further degree of freedom, such that the grading robot can lift diverting plate(s) from the conveyor. This functionality makes simplifies the task of cleaning the plates following use.
**FIG. 9h** includes a perspective view of a single degree of freedom grading robot, wherein the vertically movable support member is oriented at an angle with respect to the conveyor, so as to even further minimize the chance of damaging and/or changing the orientation and/or alignment of the other portions of the food article. For example, if the gripped portion(s) is to be moved then the chance of moving further portions is minimized by orienting the vertically movable support member at an angle, such that the gripped portion will be moved away from the further portions when it is moved from its original alignment and orientation on the conveyor.
**FIGs. 9****(i-k)** include perspective views of a four degrees of freedom grading robot. **FIGs. 9****(j-k)** specifically include perspective views of a four degrees of freedom grading robot comprising two means for moving at least one portion of a food article. In this example, the two means for moving at least one portion of a food article can rotate around an axis that is parallel to the conveyor plane. Referring to **FIGs. 9****(i-j)**, a first means for moving at least one portion of a food article grips a portion of the food article (the pinbone portion of a fish fillet in the illustration of **FIG. 9(j)****)**. Referring to **FIG. 9k**, rotation of the vertically movable support member along the fourth degree of freedom moves the first means for moving at least one portion of a food article, with the gripped portion, away from the surface of the conveyor, while also moving a second means for moving at least one portion of a food article into position to grip a further portion.

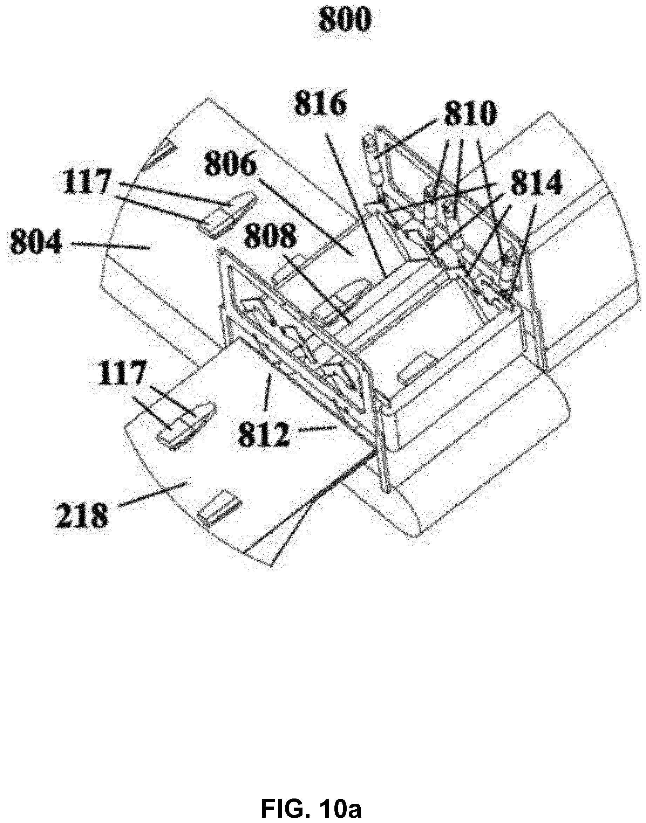

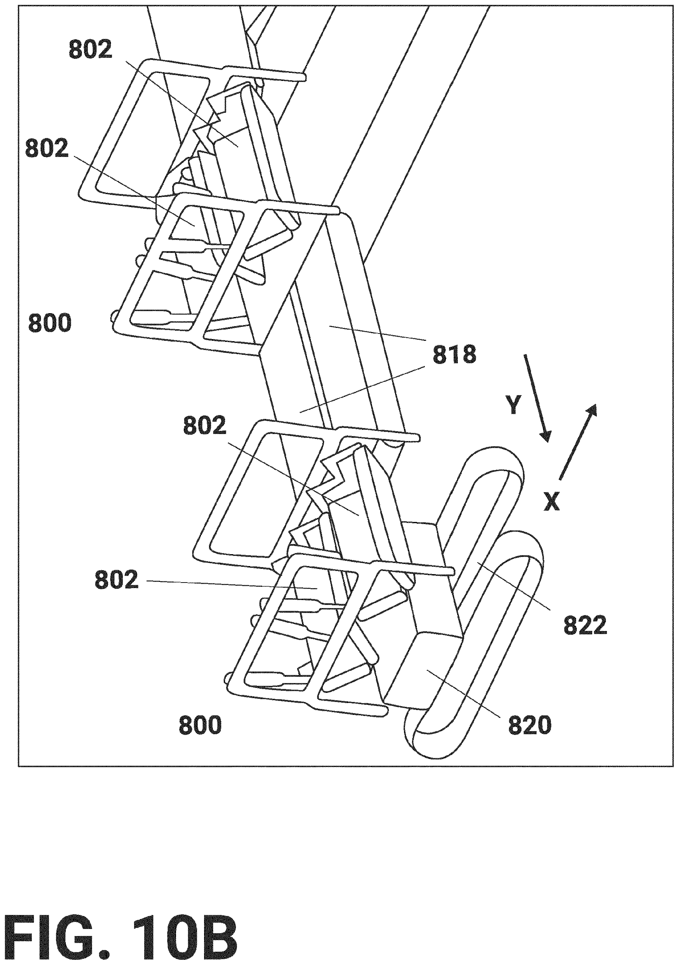

**FIG. 10****(a-b)** includes perspective views of illustrative realigning sections of a food processing apparatus. **FIG. 10a** includes a perspective view of an example of a realigning section comprising realigning apparatus. **FIG. 10b** includes a perspective view of illustrative first and second realigning sections of a food processing apparatus.

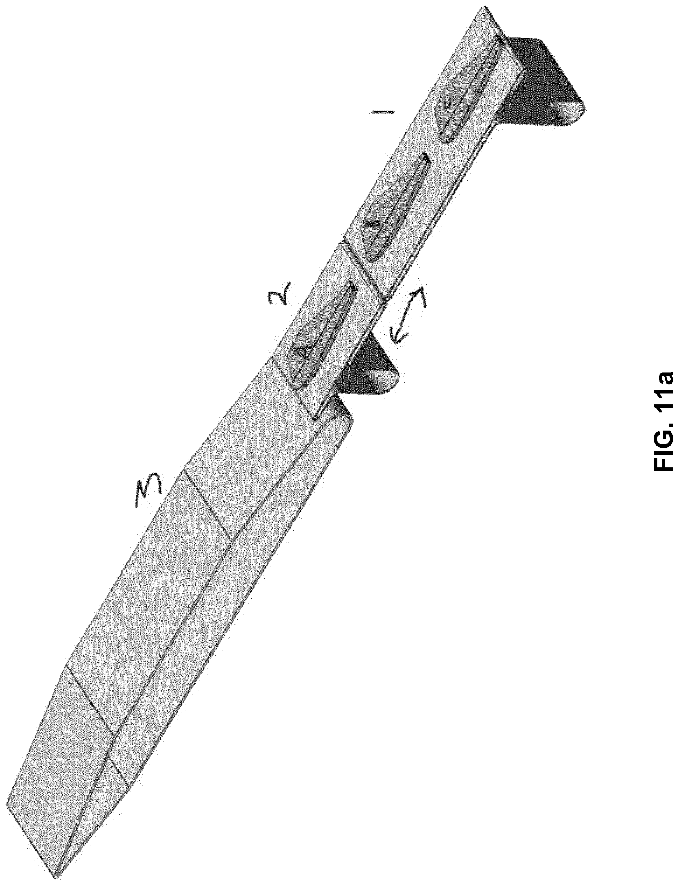

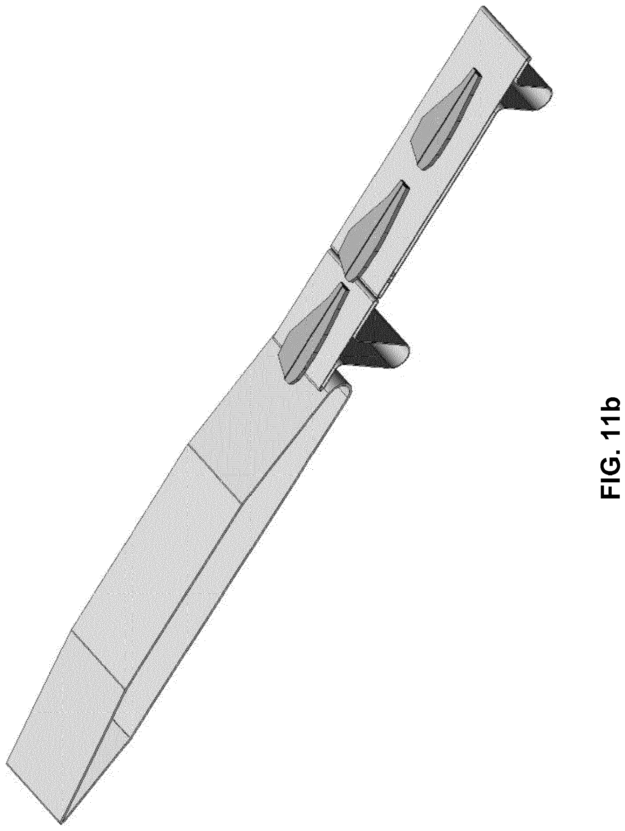

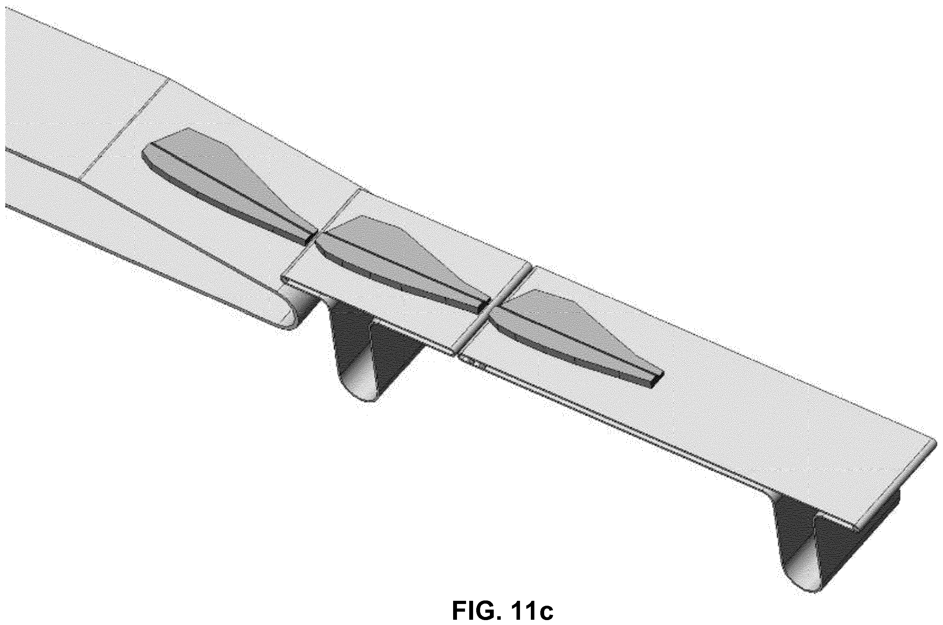

**FIG. 11** includes perspective views of a representative system that may be used to align food articles in some embodiments.
**FIG. 11****(a-c)** shows a system including three conveyors. The conveyor furthest to the left is the first conveyor (*e.g*., an x-ray conveyor) in the conveyor's direction of movement. The second conveyor from the left may be a retractable infeed conveyor. The conveyor furthest to the right may also be a retractable infeed conveyor; for example, where a person stands to ensure that food articles are properly oriented, and/or would that there is a desired spacing between the food articles (*e.g*., from overlapping ( less than 0 mm) to 100 mm, from adjacent (0 mm) to 100 mm, from about 10 mm to 100 mm, and about 10 mm). The outfeed end of the first conveyor and the infeed end of the second conveyor move together in the conveyor's direction of movement as shown with the blue arrow. There may be a product sensor (*e.g*., an imaging device) associated with the first conveyor that measures the distance between the food articles on the conveyor. For example, the product sensor may first measure the distance between consecutive food articles on the conveyor.

In the example shown in **FIG. 11(a)**, the spacing between food article A and food article B is higher *(e.g.,* more than 100 mm) than the desired spacing *(e.g.,* about 10 mm). In this case, the system will wait until food article A has reached the second conveyor, and then it will move the outfeed end of the first conveyor and the infeed end of the second conveyor at the same time, until the spacing between food article A and food article B is close to the desired spacing (*e.g*., approximately or exactly the desired spacing).

In the example shown in **FIG. 11(b)**, the desired spacing has been obtained between food article A and food article B. Once a sufficient part of food article B has been transferred over to the second conveyor, the outfeed end of the first conveyor (and the infeed end of the second conveyor at the same time) is moved back in the direction opposite to which the conveyor system is moving, such that it will be ready to move forward again for the next food article.

In the example shown in **FIG. 11(c)**, the desired spacing has been obtained between all three food articles.

### DETAILED DESCRIPTION

Disclosed generally herein are food processing systems and apparatus comprising one or more of: at least one x-ray machine to determine the location of undesirable components *(e.g.,* bones) within a food article *(e.g.,* a fish fillet); at least one cutting machine to cut a food article into portions; at least one food grading apparatus for grading portions of a food article based on different characteristics; and at least one realigning apparatus, as well as methods utilizing the same. The grading apparatus may be able to grade portions of a food article oriented parallel to each other and in series with each other with respect to the direction of movement of a conveyor surface, as is described in further detail below, and may be able to grade portions of a cut food article from the middle of the article. The grading apparatus may be able to grade portions while maintaining an original alignment and orientation of the portions.

The illustrations presented herein are not meant to be actual views of any particular food processing apparatus, x-ray machine, cutting machine, food grading apparatus, realigning apparatus, or component thereof, but are merely simplified schematic representations employed to describe illustrative embodiments. The drawings are not necessarily to scale.

Relational Terms: As used herein, any relational term, such as "first," "second," "over," "beneath," "top," "bottom," "underlying," "up," "down," etc., is used for clarity and convenience in understanding the disclosure and accompanying drawings, and does not connote or depend on any specific preference, orientation, or order, except where the context clearly indicates otherwise. The terms "vertical" and "horizontal" merely refer to a drawing figure as oriented on the drawing sheet, and in no way are limiting of orientation of a food processing apparatus, food grading apparatus, or any portion thereof.

Alignment: As used herein, the term "alignment" refers to the position of a food article and the surface of a conveyor in the vertical dimension; for example, the completeness of the contact between a food article and a conveyor surface. In particular examples, a food article may be flat, bunched, or folded. In some embodiments, whether the alignment of a food article portion during grading is "substantially preserved" may be determined by whether the food article portion(s) remains flat on the surface of the conveyor throughout the process; *e.g*., the entire bottom surface of the portion retains contact throughout the process with the surface of the conveyor.

Cutting: As used herein, the term "cutting" includes, for example and without limitation: cutting and removing unwanted components (*e.g*., bones, cartilage, fat, defects in flesh, tough tissues, skin, blood, and organs) from a food article, such as a fish fillet, and/or cutting a food article into portions. The term "advanced cut" refers specifically to the process by which a food article is cut into portions by a cutting machine.

Grading: As used herein, the term "grading" refers to a process by which portions of a food article (*e.g*., a fish fillet, cut so as to maintain the resulting portions together in the general shape of the original fillet; **FIG. 1**) are separated, for example, according to one or more selection criteria. In some embodiments, a food article is graded by a method comprising "movement" of at least one portion of the food article to an area that is separate from an area including at least one of the remaining portions of the food article. In some examples, a food article is graded by moving at least one portion of the food article to remove the portion(s) from a conveyor surface.

In series: As used herein, the term "in series" refers to portions arranged next to each other in a direction in which a conveyor is moving. In the illustrative cut food article **101** of **FIG. 1****,** portions "one," "two," "five," and "nine" are in series; *i.e.,* they are next to each other in the direction **119** the conveyor is moving.

Orientation: As used herein, the term "orientation" refers to the position of a food article and the surface of a conveyor in the two dimensions of the surface. In some embodiments, whether the orientation of food article portions during grading is "substantially preserved" may be determined by whether the position of one side (or edge) of the food article portion(s) is maintained throughout the process with respect to an edge of the conveyor. In some examples, "substantial preservation" of the orientation of food article portions may be determined by whether the general shape of the portion(s) is maintained throughout the process on the conveyor. For example, a bilaterally symmetrical portion may be flipped upside-down on the conveyor in these examples, wherein the orientation is substantially preserved, because the general shape of the portion is maintained.

Parallel: As used herein, the term "parallel" refers to portions arranged next to each other in a direction perpendicular to the direction in which a conveyor is moving. In the illustrative cut food article **101** of **FIG. 1****,** portions "two," "three," and "four" are parallel; *i.e*., they are next to each other in the direction perpendicular to the direction **119** the conveyor is moving.

Properly (aligned and/or oriented): As used herein, the term "properly aligned" refers to a food article or portion(s) thereof that is flat against a conveyor (*e.g*., without any folds) upon which the article or portion(s) is being transported, and the terms "proper orientation," "oriented properly," and "properly oriented" mean that the food article or portion(s) thereof is positioned correctly with respect to the conveyor. In other words, the terms "proper orientation and alignment," "oriented and aligned properly," and "properly oriented and aligned" mean that the shape of a food article or portion(s) thereof is positioned in a certain manner with respect to the conveyor and the direction the conveyor is moving.

Robot: As used herein, the terms "robot" and "device" are used interchangeably.

Tray: As used herein, the term "tray" refers to any receptacle for a graded portion of a food article.

**FIG. 2** is a top perspective view of a food processing apparatus **200** according to some embodiments. The food processing apparatus **200** may detect and cut undesired portions **117** from food articles, for example, being carried through the food processing apparatus **200** by the directional movement of a feed conveyor **208** surface. The food processing apparatus **200** may grade food articles according to certain characteristics (*e.g*., weight, quality, size, type, etc.). For example, the food processing apparatus **200** may be configured to automatically cut pin bones, other bones or bone fragments, cartilage, and/or other undesirable components from a food article **101**. The food processing apparatus **200** may cut the food article **101** into desired portions, **117** and grade the portions **117** according to at least one characteristic. In the drawings, the food articles are illustrated by way of example as fish fillets; however, the food processing apparatus **200** in other examples is used to process and grade any of several different food articles (*e.g*., beef, pork, poultry, lamb, crustaceans, etc.).

The food processing apparatus **200** may be utilized in the meat industry to cut ribs away from a carcass, such that the ribs are not cut, but the food processing apparatus **200** can cut through other bones in the meat sections. The food processing apparatus **200** may be utilized in the poultry industry for fully automatic detection and cutting of cartilage, for example, in the front tip of chicken breasts, which commonly remain attached to the breasts after filleting.

An exemplary food processing apparatus **200** according to some embodiments may include one or more of each of: a feed conveyor **208**; an aligning section **210**; an first x-ray machine **212, 217**; a cutting conveyor **214**; a cutting machine **216**; a first imaging system **234**; a removal or check section **244, 254**; at least a second imaging system **235**; and at least one computer.

As shown in **FIG. 2**, an exemplary food processing apparatus **200** according to some embodiments may include a feed conveyor **208**; an aligning section **210**; a first x-ray machine **212**; a cutting conveyor **214**; a cutting machine **216**; a first imaging system **234**; an automatic removal section **244**; a (*e.g*., manual) check section **254**; at least a second x-ray machine **217**; at least a second imaging system **235**; a rejection section **220**; a grading conveyor **218**; at least one grading robot **224**; at least one processing conveyor **226**; at least one realigning section **800**; at least one realigning apparatus **802**; and at least one computer. With regard to each of the foregoing components of a food processing apparatus **200** according to certain embodiments, the computers described independently for each may in some examples be either a single central computer or in other examples a plurality of computers.

As shown in **FIG. 2**, a pre-trimming line **206** may precede the food processing apparatus **200**. On the pre-trimming line **206**, fish fillets **101** may be manually or automatically trimmed prior to entering the food processing apparatus **200**. For example, on the pre-trimming line **206**, undesirable components, for example, loose bones, cartilage, parasites, blood spots, or obvious defects in flesh, may be removed from fish fillets **101**. In some embodiments, fish fillets **101** within the pre-trimming line **206** may include skin. In some embodiments, the fish fillets **101** may not include skin.

The feed conveyor **208** may follow the pre-trimming line **206**, and may transport fish fillets **101** leaving the pre-trimming line **206** to the aligning section **210**. In the aligning section **210**, fish fillets **101** may be properly aligned and placed in a proper orientation prior to continuing through the food processing apparatus **200**. For example, a fish fillet **101** may be properly oriented when a longitudinal length of the fish fillet **101** is substantially parallel to a length of a conveyor. As another non-limiting example, a fish fillet **101** may be properly oriented on a conveyor when a tail portion of the fish fillet **101** is positioned to come first as the fish fillet **101** is transported on the conveyor.

In some embodiments, alignment and orientation of fish fillets **101** may be performed manually. In some embodiments, the fish fillets **101** may be aligned and oriented automatically by an imaging device and automated robotic arm combination (e.g., a robotic arm may be utilized to rotate or push forward a food article, such as a fish fillet). Accordingly, each fish fillet **101** may be properly aligned and oriented prior to being fed into the first x-ray machine **212**. For example, **FIG. 11** shows a system that may be used to align food articles in some embodiments.

Fish fillets **101** may be fed one by one into the first x-ray machine **212**, which may capture a first x-ray image of each fish fillet **101**. The first x-ray machine **212** may also capture the precise location of bones within each fish fillet **101**. Additionally, the first x-ray machine **212** may capture the precise location of each fish fillet **101** with respect to the feed conveyor **208**. For example, the food processing apparatus **200** may include the x-ray machine described in U.S. Patent Application Publication No. 2012/0307013 A1, the disclosure of which is incorporated in its entirety herein by this reference. The first x-ray machine **212** may transfer information regarding the location of the bones within each fish fillet **101**, and the location of each fish fillet **101** with respect to the feed conveyor **208** to a computer. The feed conveyor **208** may convey the fish fillets **101** through the first x-ray machine **212**, and then off of the feed conveyor **208** and onto the cutting conveyor **214**. In some embodiments, the feed conveyor **208** and/or the cutting conveyor **214** may comprise one or more of a belt, cords, a plurality of members linked together, etc. The cutting conveyor **214** may feed the fish fillets **101** to be cut in the cutting machine **216**.

The computer may be programmed to track the movement of each fish fillet **101** as the fish fillet **101** moves through the food processing apparatus **200**. The computer may use images taken from the first x-ray machine **212**, in addition to information related to the movement of the feed conveyor **208** and cutting conveyor **214**, to determine an estimated location of each fish fillet **101** within the food processing apparatus **200**. However, some uncertainty may arise as to the precise location of each fish fillet **101** within the food processing apparatus **200**, for example, when the fish fillet **101** moves from the feed conveyor **208** to the cutting conveyor **214**. In some examples, the uncertainty is acceptable to the process. In other examples, this uncertainty is unacceptable to the process and more accuracy is needed. Therefore, particular examples may include an imaging system **234** on the cutting conveyor **214**. The imaging system **234** may capture a three-dimensional ("3D") image of each fish fillet **101**. In some examples, the imaging system **234** captures a 3D image of the fish fillet **101** as the fish fillet **101** is disposed on the cutting conveyor **214**, before the fish fillet **101** is subject to any further manipulations that may alter its alignment and/or orientation.

As shown in **FIG. 3**, the imaging system **234**, which may point towards the cutting conveyor **214**, in some embodiments may include a 3D camera **302**. The imaging system **234** may include a laser **304** that puts a light on the fish fillet **101**. In some embodiments, the imaging system **234** may capture a silhouette image or a full color 3D image. In some embodiments, the x-ray image of each fish fillet **101** may be used to generate a 3D image of the fish fillet **101**. In some examples, the intensity of each pixel in the first x-ray image may be based on the thickness of the fish fillet **101**. For example, the thinner the fish fillet **101** is, the higher the intensity of a correlating pixel may be, and conversely, the thicker the fish fillet **101** is, the lower the intensity of a correlating pixel may be. With the 3D camera **302**, it may be possible to capture a height profile of the fish fillet **101** along the image.

The 3D image of each fish fillet **101** captured by the imaging system **234** may provide an accurate measurement of a volume of each fish fillet **101**. Furthermore, a color 3D image may provide information as to a location of fat within the fish fillet **101**. The 3D image may also provide more accurate information as to the weight distribution of each fish fillet **101**. For example, the imaging system **234** may transfer the 3D image to the computer. The computer may be programmed to determine an alignment and orientation of each fish fillet **101**. From any and all of the foregoing information, the computer may adjust cutting procedures (described in further detail below), for example, to account for the particular alignment and orientation of the fish fillet **101**.

The computer may match the first x-ray image of each fish fillet **101** captured by the first x-ray machine **212** with the 3D image of each fish fillet **101** captured by the imaging system **234** *(e.g.,* by mapping the image from tail to head) to determine the precise location of the bones within each fish fillet **101** with respect to the cutting conveyor **214**. In some embodiments, this may be accomplished by matching a center of gravity of the first x-ray image and the 3D image of each fish fillet **101**, as well as the principle axis of the first x-ray image and the 3D image of each fish fillet **101**, as described in a mapping procedure of U.S. Patent Application Publication No. 2012/0307013 A1. In some embodiments, multiple x-ray images may be taken of each fish fillet **101** at differing angles, and the multiple resulting x-ray images are matched using a coordinate system, as described in U.S. Patent Application Publication No. 2012/0307013 A1. Any combination of the above described methods for determining the precise location of bones within a fish fillet **101** may be used. Furthermore, any of the mapping procedures described in U.S. Patent Application Publication No. 2012/0307013 A1 may be used.

The computer may determine an individualized cutting pattern for each fish fillet **101** using information related to the precise location of bones within each fish fillet **101**. For example, the cutting pattern of each fish fillet **101** may be determined to cut out portions **117** of the fish fillets **101** containing bones, while minimizing an amount of flesh removed with the bones. Furthermore, the cutting pattern may also be determined based on the weight distribution of the fish fillet **101** determined by the first x-ray image and 3D image. Thus, the cutting pattern can be determined to cut the fish fillets **101** into portions **117** of fixed weight, length, and/or in a pattern which optimizes a portion **117** yield. The cutting pattern for each fish fillet **101** may be superimposed onto the first x-ray image of the fish fillet **101**, 3D image of the fish fillet **101**, or both. The fish fillet **101** may then be cut into portions **117** according any of the methods and using any of the cutting machinery described in U.S. Patent Application Publication No. 2012/0307013 A1. The cutting machine **216** may include at least one cutting robot **400**, which is described in further detail below with reference to **FIG. 4**. In some examples, a single cut portion **117** of a fish fillet **101** may contain all the parts of the fish fillet **101** having bones, and another cut portion **117** may include all the parts not having bones. A fish fillet **101** may be cut into a shape that maintains the outline of the original fish fillet **101**, wherein the shape comprises both portions **117** that are in series with one another, and into portions **117** that are parallel to each other.

**FIG. 4** depicts two illustrative cutting robots **400**. However, any cutting robot (for example, any of the cutting robots described in U.S. Patent Application Publication No. 2012/0307013 A1) may be used in certain embodiments. The cutting robot **400** may be powered by any means known in the art, for example and without limitation, air cylinders, motors, and hydraulic powering means. The cutting robot **400** may also use any type of cutting known in the art, for example and without limitation, waterjet cutting, cutting with rotating knives, and ultrasonic cutting. If water jet cutting is used any type of cutting head can be used to focus the high pressure water into a thin beam that can cut through the food objects. The cutting robot **400** may also have any number of degrees of freedom. For example, a cutting robot **400** with a single degree of freedom may move in a direction perpendicular to the cutting conveyor **214**, or at an angle to the cutting conveyor **214**, such that it is capable of make straight cuts across the surface of the cutting conveyor **214**.

**FIG. 4a** illustrates a "delta" or "spider" cutting robot **401** with three degrees of freedom. If the x-axis is in the direction of movement of the cutting conveyor **214**, the y-axis is horizontal to the direction of movement of the cutting conveyor **214**, and the z-axis is the height from the cutting conveyor **214**, the "delta" or "spider" cutting robot **401** may be mounted with a base plate **402** in a plane parallel to the surface of the cutting conveyor **214**, and at a distance above the surface of the cutting conveyor **214**. The "delta" or "spider" cutting robot **401** may further include a parallel plate **404** that is parallel to the base plate **402**, where a cutting head (not shown) may be mounted. The "delta" or "spider" cutting robot **401** may further include three motors **406**, which may rotate a solid shaft **408** that is connected with bars **410** to the parallel plate **404**. The "delta" or "spider" cutting robot **401** may then move freely in the x-, y-, and z-direction within a moving envelope. In certain examples, the moving envelop may be about 200 mm in the z-direction, and about 600-800 mm in the x- and y-direction.

As illustrated in **FIG. 4b**, the cutting robot **400** may be a four degrees of freedom cutting robot **412**, where the additional degree of freedom allows the four degrees of freedom cutting robot **412** to cut a fish fillet **101** at a certain angle. In such an embodiment, the four degrees of freedom cutting robot **412** may include a motor **414** at the top of the base plate **402** that can rotate the shaft **416**. The shaft **416** may be connected to a perpendicular gear **418** that is connected to a parallel plate **404**. A cutting head **420** may be connected to the perpendicular gear **418**, such that a rotation of the cutting head **420** can be performed. In some examples, the cutting head **420** can rotate around any axis in its moving envelope that is parallel to the x-axis.

In some embodiments, the cutting robot **400** may be a six degree of freedom cutting robot (not shown). A six degree of freedom cutting robot may be able to move freely in the x-, y-, and z-direction, and also be able to rotate around the x-, y-, and z-axis to perform arbitrary cuts.

In some embodiments, during cutting procedures, an alignment and orientation of each fish fillet **101** with respect to the cutting conveyor **214** may be at least substantially preserved. In some embodiments, the alignment and/or orientation of the fish fillet **101** may be disturbed. In particular embodiments, the cutting machine **216** may include a scraper located proximate a top surface of the cutting conveyor **214**. The scraper may be so located as to contact the now cut portions **117** of the fish fillet **101** and to generally realign portions **117** that have moved out of the original alignment and orientation.

Referring to again to **FIG. 2**, the cutting machine **216** may include an automatic removal section **244**. Alternatively, the automatic removal section **244** may be located outside the cutting machine **216**, but preferably closely proximate the cutting machine **216** with the cutting robot **400**. The automatic removal section **244** may include at least one robotic arm **500** (as described in more detail below in **FIG. 6**) following the at least one cutting robot **400** of the cutting machine **216**, for removing cut portions **117** of a fish fillet **101** having bones. A robotic arm **500** of a food processing apparatus **200** may also be, for example, a grading robot **224**, as described in further detail below (*e.g*., needle grading robot **600**, two-degree of freedom grading robot **700**, three degrees of freedom grading robot, four degrees of freedom grading robot (**FIGs. 9****(i-k)**), gripper grading robot **601**, or spray-removal robot **651** (**FIG. 7a**).

In some embodiments, the cutting machine **216** communicates with the robotic arm **500**, for example, *via* a computer receiving an input signal from the cutting machine **216**, and delivering an output signal to the robotic arm **500**. For example, the cutting machine **216** may communicate to the robotic arm **500** the location, alignment, and/or orientation of portions **117** cut according to the cutting pattern employed by the cutting machine **216**, and may also communicate the identification of portions **117** containing bones or other undesirable attributes, so that such portions can be removed by the robotic arm **500**.

The removal process may be performed while the cut fish fillets **101** are still on the cutting conveyor **214**, as the most accurate information about the precise location of the portions **117** and bones is available. The computer may control the at least one robotic arm **500** and direct the at least one robotic arm **500** to grip portions **117** of a fish fillet **101** having bones, and to remove the portions **117** from the cutting conveyor **214**. For example, portions **117** of the fish fillets **101** known to have bones (*e.g*., a pin bone section) may be automatically removed by the at least one robotic arm **500**. Furthermore, portions **117** of the fish fillets **101** that are indicated as having bones in the first x-ray image or 3D image may be removed.

**FIG. 5** is a perspective view of a robotic arm **500** of the food processing apparatus **200** of **FIG. 2**. In some embodiments, at least one robotic arm **500** may grip a portion **117** of a cut fish fillet **101**, and slide the portion **117** off of a side of the cutting conveyor **214** into a first conveyor or tray **252**.

As shown in **FIG. 5**, a robotic arm **500**, located in the automatic removal section **244** and proximate the exit of the cutting machine **216**, may include a gripper **502**, a first motor **504**, a second motor **506**, a first control arm **508**, and a second control arm **510**. The first control arm **508** may be coupled to the first motor **504** at a first end of the first control arm **508**, and may be coupled to the gripper **502** at a second end of the first control arm **508**. The second control arm **510** may be coupled to the second motor **506** at a first end of the second control arm **510**, and may be coupled to the gripper **502** at a second end of the second control arm **510**. The first motor **504** and second motor **506** may be controlled by a computer. The first motor **504** may rotate the first control arm **508** about the first end of the first control arm **508**. The second motor **506** may rotate the second control arm **510** about the first end of the second control arm **510**. When the first ends of the first and second control arms **508, 510** are rotated, the second ends of the first and second control arms **508, 510** may manipulate the gripper **502**. For example, the computer may control the first and second motors **504, 506** to move the first and second control arms **508, 510,** respectively, and, in turn, manipulate the gripper **502** to grip portions **117** of a cut fish fillet **101** containing bones. The computer may further manipulate the gripper **502** to dispose the portions **117** of the fish fillet **101** containing bones in a first conveyor or tray **252**. In some embodiments, the gripper **502** is rotated with an actuator; for example and without limitation, an air cylinder, motor, linear motor, traditional motor, or solenoid (not shown in images).

In particular embodiments, the gripper **502** may include a hook that can be slipped under a portion **117** to be removed, or stuck into the portion **117** to be removed. In particular embodiments, the gripper **502** may include at least two members that can clamp or press the portion **117** between the two members. In particular embodiments, the gripper **502** may include a suction member that uses a vacuum to suck the portion **117** against the gripper **502**. In particular embodiments, the at least one robotic arm **500** may include the robotic gripper unit described in U.S. Patent Application Publication No. 2012/0307013 A1, and/or may include the associated gripper described in U.S. Patent Application Publication No. 2012/0307013 A1. In particular embodiments, the at least one robotic arm **500** may be replaced with at least one grading robot **224**, which is described above and in further detail below in relation to **FIG. 6** and **FIG. 9****.**

In some embodiments, the first conveyor or tray **252** may comprise a box, bin, tub, and/or basket. In some embodiments, the at least one robotic arm **246** may grip a portion **117** and lift the portion **117** off of the cutting conveyor **214** and subsequently place the portion **117** in the first conveyor or tray **252**. In some examples, the alignment and orientation of the portions **117** of the fish fillet **101** not containing bones are not substantially disturbed during the removal process. In other words, an alignment and orientation of the portions **117** not containing bones may be preserved, while portions **117** containing bones are removed. The removal process may be fully automatic, and may be controlled by the computer, for example, based on at least one of the first x-ray image, 3D image, and cutting pattern of each fish fillet **101**. In some embodiments, the cutting machine **216** may include a robotic arm **500** similar to the any of the robotic arms described in U.S. Patent Application Publication No. 2012/0307013 A1.

The food processing apparatus **200** may optionally include a check section **254** following the cutting machine **216** and automatic or manual removal section **244**. In some embodiments, the cutting machine **216** may not include the at least one robotic arm **246**, and the check section **254** may serve to remove portions **117** of a fish fillet **101** containing an undesirable component; for example, bones. In some embodiments including the at least one robotic arm **246**, the check section **254** may be utilized to ensure that after the fish fillet **101** has been cut, and portions **117** removed by the at least one robotic arm **246**, that all portions **117** containing bones have been removed. Portions **117** containing bones that are removed in the check section **254** and placed in a second conveyor or tray **253**. In some examples, the check section **254** is a manual check section **254**. In some embodiments, the food processing apparatus **200** may not include a check section **254**, and may include just the at least one robotic arm **246** for removing portions **117** of fish fillets **101** having bones. All portions **117** of a fish fillet **101** placed in the first and second conveyors or trays **252, 253** may then be taken to be used in other products and applications. The alignment and orientation of the remaining portions **117** may be preserved.

As shown in **FIG. 2**, a fish fillet **101** may be moved into a second x-ray machine **217**. The second x-ray machine **217** may function the same as the first x-ray machine **212**, with similar components, and may take a second x-ray image of the fish fillet **101**. The second x-ray machine **217** may x-ray and capture second x-ray images of the remaining portions **117**, which may be oriented parallel to each other, in series with each other, or both. The second x-ray machine **217** may transfer the second x-ray images to the computer. The computer may be programmed to determine, based on the second x-ray images from the second x-ray machine **217**, whether any bones remain in any of the remaining portions **117** of the fish fillet **101**. Furthermore, the computer may be programed to determine from the second x-ray images whether the fish fillet **101** has been advanced cut by the cutting machine **216**.

In some embodiments, the second x-ray images may be utilized by the computer, for example and without limitation, to control a pull-back conveyor (not pictured) that directs portions **117** that still contain bones onto another conveyor (not pictured) while the remaining portions **117** that are bone-free may move onto the grading conveyor **218**. In some examples, the bones from the portions **117** that still contain bones may be manually removed, and the manually-processed portions **117** may be moved again through the second x-ray machine **217**. By way of further example, the second images may be utilized to provide feedback to the computer controlling the cutting robot **400** about the performance of the cutting. In certain examples wherein the cutting machine **216** has a tendency to leave bones in portions that are supposed to be bone-free, this feedback is important for correcting this tendency, but the feedback may also assist in optimizing yield.

Optimization of the yield following the cutting process occurs when there is some error rate in which operation of the cutting robot **400** fails to completely cut away all of a bone or bone fragment into a portion for removal. For example, when a computer is controlling the cutting robot **400** to make the closest cut possible to the bone or bone fragment, the rate of errors is likely to increase. Conversely, when a computer is controlling the cutting robot **400** to minimize the occurrence of such errors, the amount of flesh left in portions **117** containing bones is likely to increase. Therefore, in some embodiments, the second x-ray images may be utilized to provide feedback to the computer controlling the cutting robot **400** about the error rate, and the computer controlling the cutting robot **400** may adjust the operation of the cutting robot **400** to approach an optimal error rate (*e.g*., a non-zero rate of bones and bone fragments remaining in cut portions **117**).

Further optimization of the yield can be obtained by inspecting the portions **117** containing bones that are identified by the second x-ray machine **217**, as the characteristics of the extra tissue on those portions can then be evaluated.

In some embodiments, the remaining portions **117** of the fish fillet **101** may move from the cutting conveyor **214** to the grading conveyor **218**, while at least substantially maintaining the alignment and orientation of the remaining portions **117**. The grading conveyor **218** may be larger in width than the cutting conveyor **214**, to allow for more portion positions **256** during the grading process, as described in further detail below. Furthermore, the grading conveyor **218** may include one or more sub-conveyors in series. In some embodiments, one or more sub-conveyors forming the grading conveyor **218** may vary in size and rate of movement. The grading conveyor **218** may feed the remaining portions **117** of the fish fillet **101** through a rejection section **220**. The rejection section **220** may remove an entire fish fillet **101** from the grading conveyor **218** that, for some reason, may not have been cut into portions **117** in the cutting machine **216**. For example, the rejection section **220** may include a scraper to slide an uncut fish fillet **101** off of the grading conveyor **218** and into a tray or onto a separate conveyor for further processing. The rejection section **220** may send data to a computer about an uncut fish fillet **101**, and the computer may be programmed to adjust or improve cutting procedures based on the data.

As shown in **FIG. 2**, a fish fillet **101** may be moved into to a second imaging system **235**, which may function the same as the first imaging system **234**, with similar components. For example, a laser **304** may illuminate the portions **117**, and a 3D camera **302** may transfer a 3D image to a computer programmed to determine features comprising, for example and without limitation, the location of fat, color, gaping, and other visual defects. In some examples, the second imaging system **235** captures a 3D image of a portions **117** as the portion **117** is disposed on a receiving conveyor, before the fish fillet **101** is subject to any further manipulations that may alter its alignment and/or orientation (*e.g*., a grading conveyor **218**). Using certain wavelengths, the laser **304** may provide a 3D image utilized by the computer to detect parasites. From any and all of the foregoing information, the computer may adjust grading procedures (described in further detail below), for example, to grade the portions **117** according to these features.

In some embodiments, the remaining portions **117** of the fish fillet **101** may move from the second imaging system **235** into one or more further cutting machines **216**, either directly, or after or betwixt grading procedures. In particular embodiments, the computer uses the information gathered from processing the 3D image to adjust or control cutting procedures in a further cutting machine **216**.

In embodiments, the grading conveyor **218** may feed the portions **117** of a cut fish fillet **101** through a grading section **222** of the food processing apparatus **200**. The grading section **222** may include at least one grading robot **224**, at least one tub **258** beneath the grading conveyor **218**, and a plurality of processing conveyors **226**. As the remaining portions **117** pass under the grading robot(s) **224**, the computer may direct the grading robot(s) **224** to move portions **117** of a fish fillet **101**. The grading robot **224** may be selected from the group consisting of a needle grading robot **600**, as described further below with respect to **FIG. 6**; a two-degree of freedom grading robot **700**, as described further below with respect to **FIG. 9**; a three degrees of freedom grading robot; a four degrees of freedom grading robot; a gripper grading robot **601**; and a spray-removal robot **651** (**FIG. 7a**) as described further below.

Referring to **FIG. 7a** and **FIG. 7e**, a spray-removal robot **651** may include at least one valve **661** that ejects a fluid (*e.g*., water) from a nozzle **663**, and at least one actuator (*e.g*., air cylinder, motor, linear motor **655** with at least one guide member **657**, traditional motor, and solenoid).

As shown in **FIG. 7a**, the mounting member **653** may extend up from a base member of the conveyor **665** and/or **667**, and may extend above the conveyor **665** and/or **667**. The support member **659**, to which the spray-removal robot **651** is attached, may be horizontally mounted to the mounting member **653**, with the nozzle **663** directed towards the first conveyor **665** (which may be the grading conveyor **118**). The support member **659** may be sufficiently spaced from the top surface of the conveyor **665** and/or **667**, such that a portion **117** may pass under the support member **659** and attached valve(s) **661**. The linear motor **655** may drive the sliding of valve **661** back and forth along the guide member **657** in at least substantially horizontal directions.

As shown in **FIG. 7e**, the spray-removal robot **651** may contain a spray array **664** containing a plurality of spray nozzles. For example, a spray array **664** may contain a left spray nozzle **666**, a right spray nozzle **672**, and/or a center spray nozzle **674**, wherein any of the spray array **664** may include at least one further nozzle in any of the left, right, and center positions. In the example shown in **FIG. 7e**, the spray array **664** includes a second center spray nozzle **676**. In the spray array **664**, a single spray nozzle or any combination of spray nozzles can be used in a particular application. For example, a larger number of spray nozzles may be used to remove a larger or wider portion **117** to be removed. For a smaller or more narrow portion **117** to be removed, a single nozzle might be used, or some but not all of the nozzles in the array that are oriented in the same direction (*e.g*., **674** and **676**). If the next portion **117** to be removed is similarly oriented, but also somewhat larger or wider, the left spray nozzle **666** or right spray nozzle **673** could additionally be used. For an even larger or wider portion **117** to be removed, both the left spray nozzle **666** and the right spray nozzle **673** could additionally be used.

Each of the nozzles **666, 672, 674, 676** may be connected to a respective valve. Each valve controls the flow of fluid to its respective nozzle. Each of the valves may be independently controllable such that the flow of fluid to, and ejection of fluid from, each of the nozzles **666, 672, 674, 676** is independently controllable.

Other arrangements of the nozzles **666, 672, 674, 676** are possible, and the arrangement shown in **FIG. 7e** is an example only. For example, the nozzles may be generally clustered. That is, each nozzle may be substantially adjacent to at least one other nozzle. The nozzles may be arranged in a net substantially triangular shape. The nozzles may be arranged in a net shape that substantially matches the typical net shape of the portion of the food article to be removed. The nozzles may be arranged in a net shape such that, when fluid is ejected from all nozzles, the net shape of the overall stream of fluid is larger than the net shape of the largest food portion to be removed.

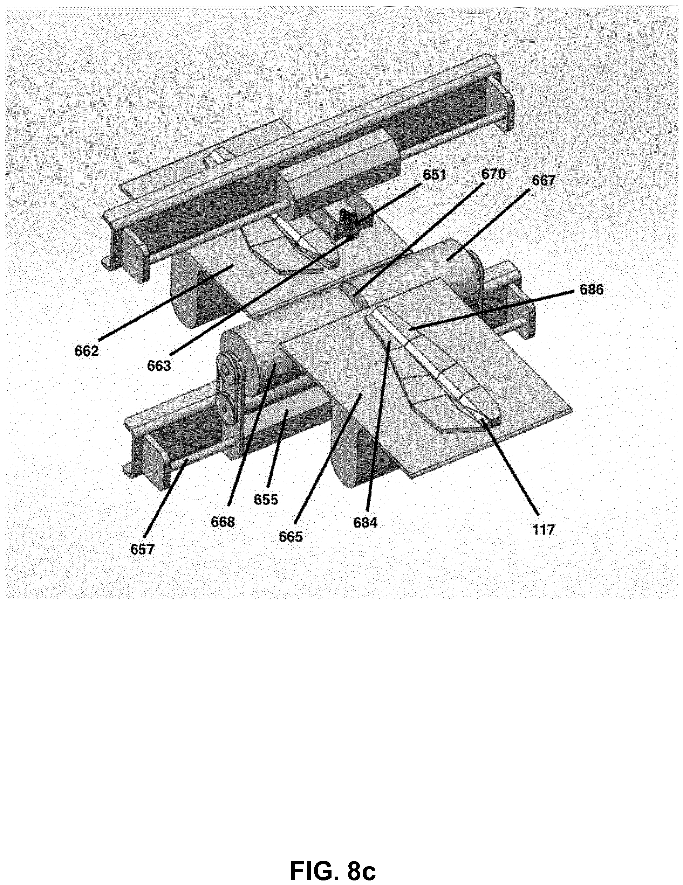

The spray array **664** described in relation to **FIG. 7e** may be combined with and implemented in any others of the arrangements described in this disclosure. For example, the spray array **664** described in relation to **FIG. 7e** may be combined with the roller system described in relation to **FIG. 8c**.

A computer may control the operation of the spray-removal robot **651**. In operation, the computer may determine a location of a portion **117** to be removed from one or more of: information from the second imaging system **235**, the second x-ray image, 3D image, first x-ray image, a signal from the cutting robot **400**, movement of the cutting conveyor **214**, and movement of the grading conveyor **218**. Based on the estimated location of the portion **117** to be removed, the computer may cause the linear motor **655** may drive the sliding of a valve **661** horizontally along the guide member **657** until the valve **661** is oriented over a projected path of the section of the portion **117** to be removed.

Once a valve **661** has been oriented over the projected path of the portion **117** to be removed, the computer may determine, for example, based on the foregoing criteria, exactly when the portion **117** will be in a location and position where the valve **661** can eject fluid through the nozzle **663** to impact the portion **117**. Then, the nozzle **663** is controlled to eject fluid along the desired path, and to shut off the fluid when the desired path is completed. In some examples, there may be multiple spray-removal robots **651** where each spray-removal robot **651** has attached a valve **661** and a nozzle **663**, and, for example, be disposed to eject fluid at the same time to impact the same section of the portion **117** to be removed, or to impact multiple sections to be removed at the same time.

According to one mode of operation, the cut portions **117** are brought by the first conveyor **665** towards the spray-removal robot **651**, and the receiving conveyor **667**. The receiving conveyor **667** may convey portions **117** thereon, for example, to an X-ray machine **217**, a further grading section **122**, a realigning apparatus **800**, or to any other conveyor where is to take place further grading, processing, and/or packaging of a fillet **101**.

When the computer determines from one or more of the second x-ray image, 3D image, first x-ray image, a signal from the cutting robot **400**, movement of the cutting conveyor **214**, and movement of the grading conveyor **218**, that the location of a section of the portion **117** to be removed (containing bones or exhibiting other undesirable attributes) is the location to be impacted by fluid ejected from the nozzle **663**, the computer may then cause the valve **661** to eject fluid through the nozzle **663**. When the section of the portion **117** to be removed is impacted by the fluid stream, it may be forced into the space between the first conveyor **665** and the receiving conveyor **667**, such that the portion **117** to be removed will not be moved to the receiving conveyor **667**, and other portions **117** of the fillet **101** are moved by the motion of the first conveyor **665** onto the surface of the receiving conveyor **667**. The spray-removal robot **651** may optionally include a laser sensor to detect the location, alignment, and/or orientation of portions **117** on the receiving conveyor **667**.

As shown in **FIG. 7b**, in particular embodiments, a spray-removal robot **651** may be used to remove portions **117** that have been cut by the cutting machine **216** at an angle **675** with respect to the first conveyor **665**. For example, the computer that controls the operation of the spray-removal robot **651** may utilize information from a laser sensor and/or preceding elements of the system **200** (*e.g.,* the cutting robot **400**) to identify a portion **117** removed, wherein the position of the portion **117** is described by a plane bisecting the portion **117** at the angle **675**. In particular examples, the portion **117** to be removed is the pinbone portion of a fish fillet **101**. In such examples, angled cuts were made to track one or more particular feature(s) in the portions **117** for removal; for example, bones, such as the pinbone of a fish fillet 1**0**1.

The ejected fluid from the spray-removal robot **651** removes the portion **117** to be removed from the remainder of the fillet **101**. In particular embodiments, the portion **117** to be removed is removed from the conveyor system by a force acting upon the section, which may be in addition to, or separate from, the force from the ejected fluid. In one embodiment, there may be sufficient space between the distal end of the first conveyor **665** and the proximal end of the receiving conveyor **667**, such that a portion **117** to be removed can be forced into the space between the first conveyor **665** and the receiving conveyor **667**, where gravity pulls the section down and away from the conveyors through the space between the distal end of the first conveyor **665** and the proximal end of the receiving conveyor **667**.

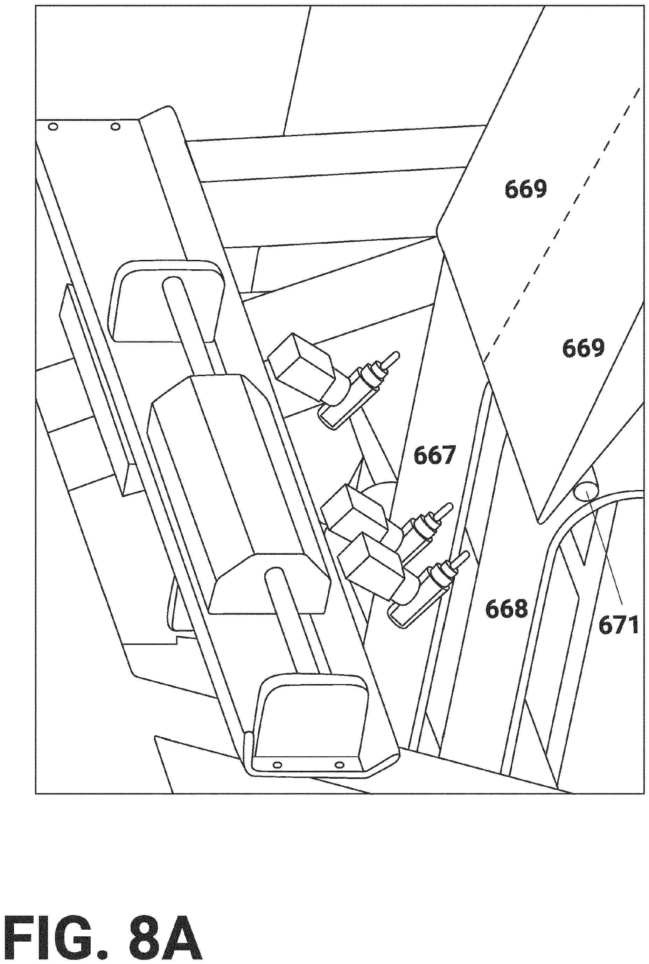

Referring to **FIG. 8a**, in one embodiment, there is a shaft **671** between the conveyors that aids in pulling down the section to be removed, for example, by increasing the space between the distal end of the first conveyor **669** and the proximal end of the receiving conveyor **667** and **668**. In some modes of operation, if the space between the distal end of the first conveyor **669** and the proximal end of the receiving conveyor **667** and **668** is increased sufficiently to aid in the removal of particular portions **117**, the transfer of remaining portions from the first conveyor **665** to the receiving conveyor **667** may by negatively impacted by the spacing; for example, the transfer may not maintain accurate location and/or alignment of portions **117**.

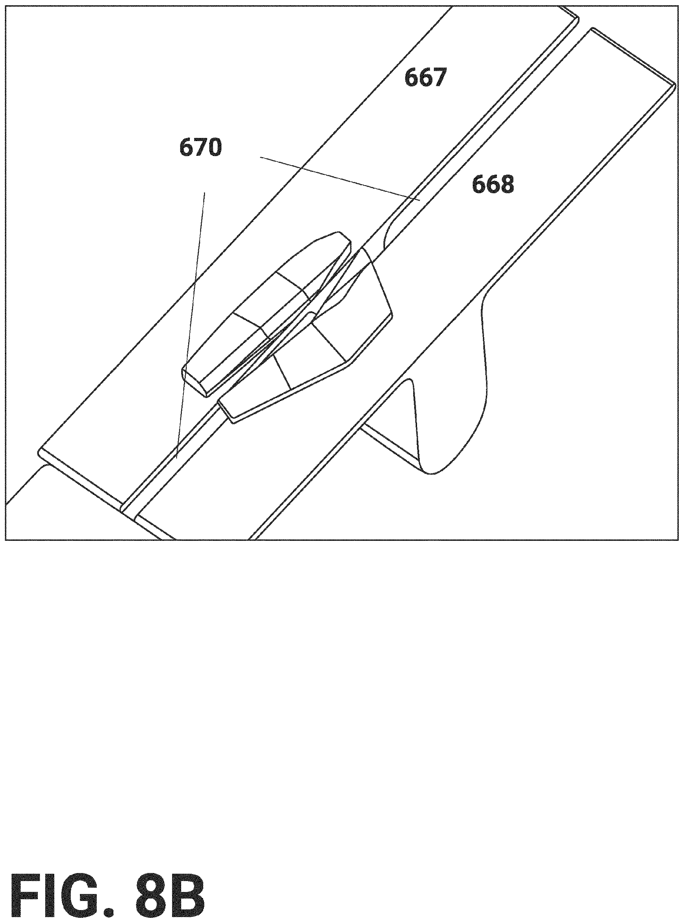

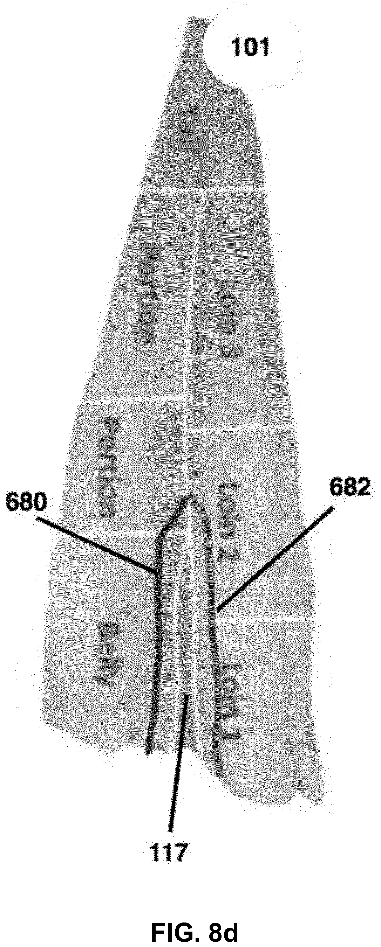

Therefore, in particular embodiments, a plate or conveyor system (**FIG. 8****(b-c)**) with a slot **670** is positioned between the first conveyor **665** and the receiving conveyor **667**, wherein the slot **670** is sized and positioned for the removal of the portion **117** impacted by the fluid from the spray-removal robot **651**. For example, the slot **670** can be of a size at least as large as the portion **117** to be removed, and the portion **117** to be removed may be moved through the slot **670**, for example, by a spray-removal robot **651**, or by an independent actuator or robot. The size of the slot **670** may be fixed, or it may be variable, controlled by an actuator.

In some modes of operation defining the size and/or position of the slot **670** between a first receiving conveyor **667** and a second receiving conveyor **668**, the first receiving conveyor **667** and the second receiving conveyor **668** may be moved in the direction perpendicular to the conveyor's movement **119**. The first receiving conveyor **667** and the second receiving conveyor **668** may be moved together in particular embodiments, or independently in other embodiments. When moved together, the slot **670** is always approximately the same width, and the first receiving conveyor **667** and the second receiving conveyor **668** may be moved, such that the slot **670** is beneath the section of the portion **117** to be removed. When the first receiving conveyor **667** and the second receiving conveyor **668** are moved independently, the width of the slot **670** (along with its position beneath the received portions **117**) can be adjusted, for example, according to information from the computer that controls the operation of the spray-removal robot **651**.

Referring to **FIG. 8c**, the plate or conveyor system may have a length of zero or a minimal length in the direction of the conveyor's movement **119**, such that the plate or conveyor system is a roller system or is essentially a roller system, respectively. Such a roller system may define the slot **670** between the first receiving conveyor **667** and the second receiving conveyor **668**, each having a circular rotation. Such a roller system may include at least one actuator (*e.g*., air cylinder, motor, linear motor **655** with at least one guide member **657**, traditional motor, and solenoid). The linear motor **655** may drive the sliding of the first receiving conveyor **667** and the second receiving conveyor **668** back and forth along the guide member **657** in at least substantially horizontal directions.

In some modes of operation, the plate or conveyor system aids in pulling down the portion **117** to be removed, for example, by controlling the size and position of the slot **670** between the first receiving conveyor **667** and the second receiving conveyor 668, such that the portion **117** to be removed falls through the slot **670** when the fillet **101** is moved by the combined action of the first receiving conveyor **667** and the second receiving conveyor **668** to a further receiving conveyor **662**. As shown in **FIG. 8c**, a fillet **101** is carried to the plate or conveyor system by the first conveyor **665**. If the portion(s) **684**, **686**, of the fillet 101 contacting the plate or conveyor system is intended to be moved to the further receiving conveyor **662**, the size of the slot **670** is reduced or minimized, or the position of the slot **670** is moved away from beneath the portion(s) **684**, **686**, to ensure that the portion(s) **684**, **686** do not fall through the slot **670**. Just before a portion **117** to be removed contacts the plate or conveyor system, the first receiving conveyor **667** and/or the second receiving conveyor **668** are moved to define the slot **670** to an appropriate size that the portion **117** to be removed falls through the slot **670**.

For example, to remove the portion **117** to be removed from the fillet **101** shown in **FIG. 8d**, the first receiving conveyor **667** may be positioned to contact the left edge **680** of the portion **117** to be removed, and the second receiving conveyor **668** may be positioned to contact the right edge **682** of the portion **117** to be removed. In some examples, the first receiving conveyor **667** and/or the second receiving conveyor **668** may be positioned to contact the fillet **101** at a suitable distance from the left edge **680** and right edge **682** of the fillet **101**, respectively, so as to define the slot **670** to be wider than the width of the portion **117** to be removed. The first receiving conveyor **667** and/or the second receiving conveyor **668** may then be controlled to follow the path of the edge of the portion **117** to be removed. Referring to **FIG. 8c**, once the portion **117** to be removed arrives in the target path of the spray-removal robot **651**, one or more spray nozzles **651** are activated to force the portion **117** to be removed through the slot **670**. Once the portion **117** to be removed has been removed through the slot **670**, the spraying may be stopped, and the location and movement of first receiving conveyor **667** and the second receiving conveyor **668** are prepared for the next fillet **101**.

In a grading section **122**, the grading robot(s) **224** may be able to remove portions **117** of a fish fillet **101** still containing bones from the grading conveyor **218** and into the at least one tub **258**. The at least one grading robot **224** may be able to move the portions **117** of a fish fillet **101** directly onto one of the plurality of processing conveyors **226**. The grading robot(s) **224** may be able to move in the direction of movement of the grading conveyor **218**, so that the grading robot(s) **224** remain above the portions **117** while the movement of the portions **117** is carried out.

The plate or conveyor system described in relation to **FIG. 8c**, for example, may be combined with and implemented in any others of the arrangements described in this disclosure. For example, the roller system described in relation to **FIG. 8c** may be combined with and implemented with the spray array **664** described in relation to **FIG. 7e**.

In some embodiments, the at least one grading robot **224** may be capable of moving to a side of the grading conveyor **218**, such that the grading robot **224** is not directly above the grading conveyor **218** but rather, may be directly above one of the plurality of processing conveyors **226** or the at least one tub **258**. In some embodiments, a top surface **262** of at least one of the plurality of processing conveyors **226** may be at least substantially coplanar with a top surface **264** of the grading conveyor **218**. In particular embodiments, the alignment and orientation of the portions **117** of a fish fillet **101** may be substantially preserved when a grading robot **224** moves the portions **117** from the grading conveyor **218** to a processing conveyor **226** having a top surface **262** substantially coplanar with the top surface **264** of the grading conveyor **218**. In particular embodiments, a top surface **262** of the processing conveyor **226** may be lower than the top surface **264** of the grading conveyor **218**, such that, when moved, the portions **117** are slid off the grading conveyor **218** by a grading robot **224** and dropped onto the processing conveyor **226**.

In some embodiments, a fish fillet **101** may be cut and portions **117** of the cut fish fillet **101** graded at the same time. In particular embodiments, the grading is performed simultaneously as part of the cutting process. For example, the cutting robot **400** may be the same robot as the grading robot **600**.

In some embodiments, a grading robot **224** may organize the portions **117** of a fish fillet **101** to different areas (portion positions **256**) of the grading conveyor **218** according to different characteristics of the portions **117**. For example, in some embodiments, a grading robot **224** may be controlled to move the tail portion of a fish fillet **101** to a certain portion position **256** of the grading conveyor **218**, and to move all of the loin portions of the fish fillets **101** to another portion position **256** of the grading conveyor **218**. In some embodiments, a grading robot **224** may organize the portions **117** of a fish fillet **101** according to a weight of the portions **117**. For example, the grading robot **224** may be controlled to move all portions **117** having a weight within a first range of weight to a certain portion position **256** of the grading conveyor **218**, and optionally to move all portions **117** having a weight within a second range of weight to another portion position **256** of the grading conveyor **218**. In some embodiments, the at least one grading robot **224** may organize the portions **117** of a fish fillet **101** according to one or more metric and/or criterion of size, type, or quality. The different portion positions **256** of the grading conveyor **218** may be designated as leading to different processes. For example, the different portion positions **256** of the grading conveyor **218** may lead to different processing conveyors **226** or trays, which in turn, may lead to any of multiple different processes. Such processes may include one or more of packaging, breading, freezing, further cutting, and any other known process for food processing.

In some embodiments, a grading robot **224** may be able to move one or more portion(s) **117** from a group of portions **117** that are oriented parallel to each other without disturbing the alignment and orientation of the other portions **117** in the group. For example, with reference to **FIG. 1**, the grading robot **224** may be able to move portion **117** two from portions **117** four and five, without disturbing the alignment and orientation of portions **117** four and five. In particular embodiments, the alignment and orientation of the cut portions **117** may be substantially preserved when the grading robot **224** moves the portions **117** from the grading conveyor **218**. The substantial preservation of orientation and/or alignment of portions during grading may provide an advantage over previously known grading robots that are limited to grading portions **117** that are in series. For example, previously known grading robots, such as scrapers, move across a conveyor and remove any portions **117** along a path of the scraper. Thus, previously known grading robots are unable to grade a single portion **117** of a group of portions **117** that are oriented parallel to each other.

At least one of the grading robot(s) **224** may be able to move a portion **117** of a fish fillet **101** without damaging the delicate flesh of the portions **117**. For example, at least one grading robot **224** may be a needle grading robot **600** as described in **FIG. 6**, which includes at least one needle **602** that can be inserted into the flesh of each portion **117** without damaging the flesh. A computer, based on one or more of the second x-ray image, 3D image, first x-ray image, and estimated location of the portion **117** following the activity of the cutting robot **400**, may control the needle grading robot **600** to a location in line with a center area of a portion **117** to be moved. The computer may then control the needle grading robot **600** to lower the needle(s) **602** into the center area of the portion **117** to be moved. The needle grading robot **600** may then be directed to move the portion **117** to a portion position **256**, by moving the needle(s) **602** to the portion position **256** without retracting the needle(s) **602** from the portion **117**, according to the selected function of the needle grading robot **600**.

Having at least one needle **602** to puncture the portions **117** may provide an advantage over previously known grading robots. For example, previously known robots used to grade delicate food products often damage the flesh by breaking, crushing, or tearing the flesh of the portion **117** while moving the portion **117**, for example, by pressing the flesh between two members. On the other hand, by puncturing the portions **117** with at least one needle **602**, the flesh may not be broken, crushed, torn, or otherwise damaged. Furthermore, using at least one needle **602** to puncture the portions **117** may allow the alignment and orientation of the portions **117** to be maintained during grading. Contrariwise, pressing the portions **117** between two members can cause folds in the flesh that can disrupt the alignment or orientation of the portions **117**.

In some embodiments, the alignment and orientation of the portions **117** may have to be maintained during the grading process for subsequent processes such as, for example, packaging and freezing processes. Accordingly, moving the portions **117** with at least one needle **602** may also provide an advantage over moving the portions **117** with scrapers, which are often used to move portions **117** of food articles **101** on a conveyor by placing a scraper to one side of the portion **117**, and pushing the portion **256** to a desired location. Using a scraper to move the portions **117** can also cause folds in the flesh, which can disrupt the alignment and orientation of the portions **117**. On the other hand, using at least one needle **602** to move the portions **117** may help to maintain the alignment and orientation of the portions **117** for further processes. For example, multiple needles **602** may be used to puncture a portion **117** and the multiple needles **602** may be spaced throughout the portion **117**. Having multiple needles **602** contacting multiple locations throughout the portion **117** while the portion **117** is moved may assist in maintaining the alignment and orientation of the portion **117** for further processes.

Referring again to **FIG. 6**, a needle grading robot **600** may include a first mounting member **603**, a second mounting member **604**, a first actuator (*e.g*., air cylinder **606**, motor, linear motor, traditional motor, and solenoid), a second actuator **608**, a first guide member **610**, a second guide member **612**, a horizontally movable support member **614**, a vertically movable support member **616**, a laser sensor 6**1**8, at least one needle **602**, and a release mechanism **620**.

The first mounting member **603** and second mounting member **604** may extend up from a base member **622** of the grading conveyor 2**1**8, and may extend above the grading conveyor **218**. The first guide member **610** may be horizontally mounted to the first mounting member **603** and the second mounting member **604**. The first guide member **610** may be sufficiently spaced from the top surface of the grading conveyor **218**, such that a portion **117** may pass thereunder. A length of the first guide member **610** may be longer that than a width of the grading conveyor **218**, such that the first guide member **610** extends out past the first and second mounting members **603**, **604**, and extends out past side surfaces of the grading conveyor **218** to allow for a grading robot **224** to place a portions **117** directly onto processing conveyors **226** next to the grading conveyor **218**, as discussed above.

The horizontally movable support member **614** may be slidable back and forth along the first guide member **610** in at least substantially horizontal directions. The second guide member **612** may be mounted to the horizontally movable support member **614**. The vertically movable support member **616** may be slidable up and down along the second guide member **612** in at least substantially vertical directions.

The first air cylinder **606** may be attached at one end to the horizontally movable support member **614**, and may be attached at another end to the second mounting member **604**. The second air cylinder **608** may be attached at one end to the second guide member **612**, and attached at another end to the vertically movable support member **616**. At least one needle **602** may be attached to a lowermost portion **624** of the vertically movable support member **616**, and a length of the needle(s) **602** may be oriented in a direction at least substantially normal to the top surface of the grading conveyor **218**. In some examples, the needle(s) **602** may have an outer diameter, for example and without limitation, within a range of 0.5 mm to 2 mm; or within a range of 0.1 mm to 0.4 mm; within a range of 2 mm to 5 mm. The release mechanism **620** may be attached to the lowermost portion **624** of the vertically movable support member **616** proximate at least one needle **602**. The laser sensor **618** may be disposed proximate the second mounting member **604**, and may track the position of the second guide member **612** and vertically movable support member **616**.

A computer may control the operation of the at least one grading robot **224**. In operation, the computer may determine an estimated location of a portion **117** to be moved based on one or more of the second x-ray image, 3D image, first x-ray image, movement of the cutting conveyor **214**, and movement of the grading conveyor **218**. Based on the estimated location of the portion **117** to be moved, the computer may cause the horizontally movable support member **614** to move horizontally until at least one needle **602** attached to the lowermost potion of the vertically movable support member **624** is oriented over a center of a projected path of the portion **117** to be moved. The laser sensor **618** may be used to determine when the vertically movable support member **616** is correctly oriented over the center of the projected path of the portion **117**. The horizontally movable support member **614** is moved, for example, by pumping air into or sucking air out of a first air cylinder **606**. By pumping air into or sucking air out of the first air cylinder **606**, the first air cylinder **606** may be caused to extend or retract. By extending or retracting the first air cylinder **606**, the first air cylinder **606** may push or pull the horizontally movable support member **614** along the first guide member **610**. By pushing or pulling the horizontally movable support member **614**, the at least one needle **602** may be moved along the first guide member **610** and across the width of the grading conveyor **218**.

Once at least one needle **602** has been oriented over the center of the projected path of the portion **117** to be moved, the computer may determine, for example, based on the estimated location of the portion **117** and based on the movement of the grading conveyor **218**, exactly when the portion **117** will be directly underneath the needle(s) **602**. The computer may then cause the vertically movable support member **624** to move downwards towards the portion **117** such that the needle(s) **602** penetrates at least partially the flesh of the portion **117** to be moved. The vertically movable support member **624** is moved downwards by the action of a second actuator, for example, by pumping air into a second air cylinder **608** and extending the second air cylinder **608**. Once the at least one needle **602** has penetrated the flesh of the portion **117** to be moved, that portion **117** can be moved by moving the horizontally movable support member **614** according to the above described procedure.

After the portion **117** has been moved by a needle grading robot **600**, at least one needle **602** may be retracted from the flesh of the portion **117**. In some embodiments, the procedure may happen naturally, for example, when the portions **117** are dropped into a tub **258**. In such examples, the portions **117** may naturally fall of the needle(s) **602**. In some embodiments, a release mechanism **620** may be used to press against the portion **117** as the at least one needle **602** is lifted. At least one needle **602** may be lifted, for example, by sucking air out of a second air cylinder **608** and retracting the second air cylinder **608**. By retracting the second air cylinder **608**, the vertically movable support member **624**, to which the needle(s) **602** is attached, may be pulled up along the second guide member **612** by the second air cylinder **608**. Once the vertically movable support member **624** has been moved up and the at least one needle **602** retracted from the portion **117**, the grading process may begin again and may repeat the above described process in relation to another portion **117** to be moved.

In some embodiments, a needle grading robot **600** may include a plurality of needles **602** oriented linearly in a direction parallel to the direction the grading conveyor **218** is moving. In some embodiments, a needle grading robot **600** may include a plurality of needles **602** oriented linearly in a direction perpendicular to the direction the grading conveyor **218** is moving. In some embodiments, a needle grading robot **600** may include a plurality of needles **602** oriented in a shape of one or more of, for example and without limitation, a square, circle, triangle, or cross. For example, the orientation of the plurality of needles **602** may be chosen based on a projected function of a specific needle grading robot **600**. For example, based on the shape of a portion **117** to be moved, certain orientations of the plurality of needles **602** may be better suited to maintain the alignment and orientation of the portion **117**.

In some embodiments, where the needle grading robot **600** includes a plurality of needles **602**, the plurality of needles **602** may all be pointed in a direction substantially normal to the top surface of the grading conveyor **218**. In some embodiments, the plurality of needles **602** may be pointed in directions not substantially normal to the top surface of the grading conveyor **218**. For example, the plurality of needles **602** may be pointed in directions, such that if the plurality of needles **602** touched the top surface of the grading conveyor **218**, an angled formed between each needle **602** of the plurality of needles **602** and the top surface of the grading conveyor **218** would be an acute angle. In some embodiments, the plurality of needles **602** may include crossing needles **602**.

In some embodiments, a grading robot **224** may lift a portion **117** from the top surface of the grading conveyor **218** in order to place the portion **117** in a portion position **256** on the grading conveyor **218**, or to lift the portion **117** from the top surface of the grading conveyor **218** to remove the portion **117** from the grading conveyor **218**, for example and without limitation, into a tray or bin or onto another conveyor entirely. In particular embodiments, a grading robot **224** that lifts a portion **117** from the top surface of the grading conveyor **218** may comprise a gripper (a "gripper grading robot" **601**). As described above with regard to a robotic arm **500**, a gripper may include, for example and without limitation, a hook that can be slipped under a portion **117** to be removed, or stuck into the portion **117** to be removed, at least two members that can clamp or press the portion **117** between the two members, a suction member that uses a vacuum to suck the portion **117** against the gripper, and/or any gripper described in U.S. Patent Application Publication No. 2012/0307013 A1.

As shown in **FIG. 6(b)**, a needle grading robot **600** may be adapted to lift a portion **117** from the top surface of the grading conveyor **218** in order to place the portion **117** in a portion position **256** on the grading conveyor **218**, or to lift the portion **117** from the top surface of the grading conveyor **218** to remove the portion **117** from the grading conveyor **218**. **FIG. 6(c)****-(d)** show at least one needle **602** attached to a lowermost portion **624** of the vertically movable support member **616** according to particular embodiments. In the needle grading robot **600** of **FIG. 6(c)**, the at least one needle **602** is disposed in eight sets of three needles **602**, wherein two sets of three needles **602** are disposed in an area of the distal surface of the lowermost portion **624** of the vertically movable support member **616**, and the needles **602** are positioned proximate the vertically movable support member **616**. In the needle grading robot **600** of **FIG. 6(c)**, the distal surface of the lowermost portion **624** of the vertically movable support member **616** is separated into four areas.

To grip a portion **117** to slide or lift the portion **117**, the needles **602** are moved to a position distal the vertically movable support member **616**, piercing the flesh of the portion **117**, as shown in **FIG**. **6(d)**. Any method may be used to extend the needles to a position distal the vertically movable support member **616**, for example, air compression. The sets of needles **602** shown in **FIGs**. **6(c)****-(d)** may be moved separately in some examples, according to the grading application. For example, to move small portions **117** only needle **602** set "one" may be extended; to move a bigger, longer portion **117**, both needle **602** sets "one" and "two" may be extended; and to move a shorter, wider portion **117**, both needle **602** sets "one" and "three" may be extended; and to move a longer, bigger portion **117**, all of needle **602** sets "one," "two," "three," and "four" may be extended.

In some embodiments, a needle grading robot **600** or gripper grading robot **601** may be capable of rotation in a plane parallel to the surface of the grading conveyor **218**, for example, to orient or reorient a portion **117** on the surface of the grading conveyor **218**. For example, as shown in **FIG**. **4** with regard to a cutting robot **400**, the needle grading robot **600** or gripper grading robot **601** may include a motor **414** that can rotate, directly or indirectly, the vertically movable support member **616**. Thus, in some examples, the vertically movable support member **616** can rotate around any axis in its moving envelope that is parallel to the x-axis (*e.g*., the surface of the grading conveyor **218**).

In particular embodiments, a needle grading robot **600** (*e.g.,* as shown in **FIGs**. **6(c)****-(d)**) may be utilized to lift a plurality of portions **117**, for example, before they are moved to a different position on the top surface of the grading conveyor **218**, or removed from the top surface of the grading conveyor **218**. Such embodiments may in some examples provide the particular advantage of advantage to increasing capacity, for example, when the plurality of portions **117** are removed from the top surface of the grading conveyor **218**. In some examples, a single needle grading robot **600** may be utilized to lift a plurality of portions **117** of a fish fillet **101** that each contain, for example, a bone or bone fragment. For example and without limitation, and with reference to **FIG.** 1, a single needle grading robot **600** may be utilized to lift portions 117 "ten" and "twelve." In this example, both needle **602** sets "one" and "two" may be utilized to grip portion **117** "ten," and needle **602** set "four" may be utilized to grip portion **117** "twelve." For example and without limitation, the needle grading robot **600** may be moved to the desired position (*e.g*., a different position on the top surface of the grading conveyor **218**, or a position that is removed from the top surface of the grading conveyor **218**), and both pieces may be released from the gripper, either individually, or at the same time. By way of further example, the needle grading robot **600** may be moved to a first desired position of a first portion **117** *(e.g.,* a different position on the top surface of the grading conveyor **218**), the first portion **117** may be released from the gripper at the first position, the needle grading robot **600** may then be moved to a second desired position of a second portion **117**, and the second portion **117** may be released from the gripper at the second position (*e.g*., a position that is removed from the top surface of the grading conveyor **218**).

By lifting the portion **117** from the top surface of the grading conveyor **218**, the grading robot(s) **224** may maintain an orientation of the portion **117** while moving the portion **117** to another conveyor. For example, moving a portion **117** from the grading conveyor **218** to a processing conveyor **126**, wherein the top surface **164** of the grading conveyor **118** is substantially coplanar with the top surface **162** of the processing conveyor **126**, sliding the portion **117** along the top surfaces of the grading conveyor **218** and another conveyor, may subject the portion **117** to conveyors traveling in different directions when the portion **117** is moved from the grading conveyor **218** to the other conveyor. Accordingly, the portion **117** is likely to at least partially rotate such that the orientation of the portion **117** is disturbed. Thus, by lifting the portion **117**, a change in orientation may be avoided.

In some embodiments, at least one grading robot **224** may slide the portion **117** along the top surface of the grading conveyor **218** to a portion position **256** on the grading conveyor **218**, as described above. In some embodiments, the grading robot(s) **224** may slide the portion **117** completely off of the top surface of the grading conveyor **218** and into a tray or tub **258** or onto another conveyor, as described above. In particular embodiments, a grading robot **224** that slides a portion **117** along the top surface of the grading conveyor **218** to a portion position **256** on the grading conveyor **218** may be a two degrees of freedom grading robot **700**.

As shown in **FIG. 9a**, an illustrative two degrees of freedom grading robot **700** according to some embodiments may include a horizontal actuator, for example and without limitation, an air cylinder **702**, a plate **704**, and a sensor **706**. The horizontal air cylinder **702** may be capable of movement in arbitrary positions in the direction perpendicular to the direction of movement of the grading conveyor **218**. The sensor **706** provides feedback on the actual horizontal position of the plate **704** to a computer. Based on the estimated location of the portion **117** to be moved, the computer may cause the horizontal air cylinder **702** to move the plate **704** to push the portion **117** to another portion position **256** on the grading conveyor **218**, or off of the grading conveyor **218**, for example and without limitation, into a tray or bin or onto another conveyor entirely. The pushing plate **704** may be so thin that it can potentially be used to go between certain portions **117** when there are more than one piece parallel on the belt.

In some embodiments, the grading section **122** of the food processing apparatus **100** may include a separating block at any location within the grading section **122**. In some embodiments, the separating block may have a triangular shape, and may be suspended above the top surface **164** of the grading conveyor **118**, but may be suspended close enough to the top surface **164** of the grading conveyor **118** for the portions **117** of a fish fillet **101** to contact the separating block. In embodiments where the separating block has a triangular shape, the separating block may be pointed (a tip of the triangle pointed) in a direction opposite to the direction in which the grading conveyor **118** is moving. Thus, when a portion **117** of a fish fillet **101** contacts the separating block, the portion **117** may slide along a side of the separating block, and may be moved in a direction perpendicular to the direction in which the grading conveyor **118** is moving and toward an outside edge of the grading conveyor **118**, until the portion **117** reaches a base of the separating block. Thus, the separating block may serve to separate portions **117** that are oriented parallel to each other.

In some embodiments, the base of the separating block may have a width less than the width of the grading conveyor **118**, such that the separating block may serve to move the portions **117** to different areas of the grading conveyor **118**. In some embodiments, the grading section **122** may include secondary conveyors disposed to either side of the grading conveyor **118** in conjunction with a separating block. In such embodiments, the base of the separating block may have a width that is equal to or wider than the width of the conveyor belt, such that when a portion **117** of a fish fillet **101** contacts the separating block, the portion **117** is moved off of the grading conveyor **118** and onto one of the secondary conveyors. In some embodiments, the secondary conveyors may move at a rate faster than the grading conveyor **118** to further separate the portions **117** from each other in a direction parallel to the direction in which the secondary conveyors are moving.

In some embodiments, the grading section **222** may include multiple grading robots **224** that perform different grading functions. For example, a first grading robot **224** may remove portions **117** that contain bones into a tub **258**, and a second grading robot **224** may, for example, grade the remaining portions **117** according to type, such as tail portions or loin portions. Furthermore, a third grading robot **224** may, for example, grade each type of portion **117** according to weight. Thus, each portion **117** may be graded according to multiple characteristics. In some embodiments, multiple grading robots **224** may be used to perform a single grading function. For example, multiple grading robots **224** may be used to remove portions **117** having bones, while other multiple grading robots **224** are used to organize the portions **117** according to another characteristic of the portions **117**.

In some embodiments, a first group of grading robots **224** may be located on a first sub-conveyor of the grading conveyor **218** and may be controlled by a computer to perform a first function, and a second group of grading robots **224** may be located on a second subsequent sub-conveyor of the grading conveyor **218** and may be controlled by a computer to perform a second function. In particular embodiments, the first sub-conveyor and second sub-conveyor of the grading conveyor **218** may have different widths to accommodate the first and second functions, respectively. In particular embodiments, the grading conveyor **218** may include additional sub-conveyors with respective groups of grading robots **224** that perform yet other functions. In some examples, each sub-conveyor of the grading conveyor **218** may have a plurality of processing conveyors **226** associated with the sub-conveyor.

In some embodiments, the alignment and/or orientation of a portion **117** may need to be changed or adjusted to allow for further processing. For example, for processes having in-feeds perpendicular to a direction in which the grading conveyor **218** is moving ("perpendicular in-feeds"), the portion **117** may need to be oriented such that a length of the portion **117** is oriented perpendicular to a direction in which the perpendicular in-feeds are moving, as shown in **FIG. 2**. In particular embodiments, the food processing apparatus **200** may include at least one realigning section **800** for each process that needs realignment for the process. A realigning section **800** may include at least one realigning apparatus **802**. A realigning apparatus **802** is described in further detail in relation to **FIG. 10**.

**FIG. 10a** is a top perspective view of a realigning section **800** according to some embodiments. As shown in **FIG. 8**, the realigning section **800** may include at least one realigning apparatus **802**. The realigning apparatus **802** may be disposed at an end of the grading conveyor **218**, and above a perpendicular in-feed processing conveyor **804**. The realigning apparatus **802** may include a first mini-conveyor **806**, a second mini-conveyor **808**, and at least one actuator (*e.g*., air cylinder **810**, motor, linear motor, traditional motor, and solenoid). The first mini-conveyor **806** and second mini-conveyor **808** may be oriented at an acute angle to each other, such that the first mini conveyor **806** and the second mini conveyor **808** form a V-shape. The first mini-conveyor **806** and second mini-conveyor **808** may be mounted above the perpendicular in-feed processing conveyor **804**, such that a length of each of the first mini-conveyor **806** and second mini-conveyor **808** is at least substantially perpendicular to a length of the perpendicular in-feed processing conveyor **804**, and the direction in which the perpendicular in-feed processing conveyor **804** is moving portions **117**.

When the first mini-conveyor **806** and second mini-conveyor **808** are oriented such that the first and second mini-conveyors **806, 808** form a V-shape, a point **816** of the V-shape may be lower than the top surface of the grading conveyor **218**, such that portions **117** may come off of the grading conveyor **218** and drop through an open end **812** of the V-shape and into the V-shape formed by first and second mini-conveyors **806**, **808**.

One or more of the first mini-conveyor **806** and second mini-conveyor **808** may be mounted above the perpendicular in-feed processing conveyor **804** with a hinge member **814**, such that the surface of the first mini-conveyor **806** and the surface of the second mini-conveyor **808** may be separated to provide a gap through which a portion **117** may fall. For example, the first mini-conveyor **806** may be mounted above the perpendicular in-feed processing conveyor **804** with a hinge member **814**, such that the first mini-conveyor **806** may swing away from the second mini-conveyor **808** and provide a gap through which a portion **117** may fall. In some embodiments, the second mini-conveyor **808** may be mounted above the perpendicular in-feed processing conveyor **804** with a hinge member **814**, such that the second mini-conveyor **808** may swing away from the first mini-conveyor **806** and provide a gap through which a portion **117** may fall. In particular embodiments, both the first mini-conveyor **806** and the second mini-conveyor **808** may be mounted above the perpendicular in-feed processing conveyor **804** with a hinge member **814**, such that the first mini-conveyor **806** may swing away from the second mini-conveyor **808**, and the second mini-conveyor **808** may swing away from the first mini-conveyor **806** to provide a gap through which a portion **117** may fall.

In operation, for example, a portion **117** may have a width and a length, and the length of the portion **117** may be longer than the width. For some processes subsequent to grading, it may be necessary to orient the portion **117**, such that the length of the portion **117** is at least substantially perpendicular to the direction in which the processing conveyor **804** is moving. To ensure that the portion **117** is oriented correctly, the first mini-conveyor **806** and second mini-conveyor **808** may be oriented to form the V-shape, in order to receive a portion **117** from the grading conveyor **218**. A grading robot **224** may organize the portion **117**, such that the portion **117** will be dropped through the open end **812** of the V-shape, and into the V-shape formed be the first mini-conveyor **806** and second mini-conveyor **808**. Belts on the first mini-conveyor **806** and second mini-conveyor **808** may rotate to receive the portion **117**, and to spread the portion **117** along the first mini-conveyor **806** and second mini-conveyor **808**. The V-shape formed by the first mini-conveyor **806** and second mini-conveyor **808** may tend to urge the portion **117** towards the point **816** of the V-shape, and to orient the portion **117** linearly along a length of the first mini-conveyor **806** and second mini-conveyor **808**. Furthermore, the V-shape formed by the first mini-conveyor **806** and second mini-conveyor **808** may tend to orient the length of the portion **117**, such that the length of the portion **117** is substantially parallel to the lengths of the first mini-conveyor **806** and second mini-conveyor **808**, and at least substantially perpendicular to the length of the perpendicular in-feed processing conveyor **804** and the direction in which the perpendicular in-feed processing conveyor **804** is moving portions **117**. Thus, when a gap is formed between the first mini-conveyor **806** and second mini-conveyor **808**, the portion **117** will drop onto the perpendicular infeed processing conveyor **804** with the length of the portion **117** oriented substantially perpendicular to the direction in which the perpendicular in-feed processing conveyor **804** is moving portions **117**. As discussed above, orienting the lengths of portions **117** to be perpendicular to the direction in which the perpendicular in-feed processing conveyor **804** is moving portions **117** may be necessary for subsequent processes such as packaging and freezing processes.

As shown in **FIG. 10b**, a food processing apparatus **200** according to some embodiments may include at least one additional realigning section **800**. In particular embodiments, the food processing apparatus **200** comprises at least one additional realigning section **800** to realign a portion when the capacity of the first realigning section **800** is exceeded. A food processing apparatus **200** may include one or more transfer conveyor **818** that directs a portion **117** to the next realigning section **800**. A next realigning section may, for example and without limitation, realign a portion **117** onto a next processing conveyor (not shown), or realign a portion **117** into a container **820** that the portion **117** is to be packed in. A food processing apparatus **200** may also include a packing conveyor **822** that can be used to move the container **820** to the correct location to receive the portion **117**. In some examples, the packing conveyor **822** moves such that the portion **117** is placed at a specific x-coordinate in the container **820**. In some examples, the time at which the realigning apparatus **802** is opened is controlled, such that a specific y-position of the portion **117** in the container **820** is selected. The container **820** may either be filled completely, or partially filled. For example, the container **820** may be partially filled, and a grader (not shown) may be used to fill container **820** completely.

While embodiments of the invention has been illustrated and described in detail in the drawings and foregoing description, such illustration and description are to be considered illustrative or exemplary and not restrictive; the invention is not limited to the particularly described examples. Other variations to the particularly described examples are understood and can effected by those skilled in the art, from a study of the drawings, the specification, and the appended claims. In the specification and claims, the terms "comprising" and "including" are open-ended, and do not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as *via* the Internet or other wired or wireless telecommunication systems. Any reference signs in the claims should not be construed as limiting the scope.

## Claims

1. A system for grading a food article (101) that has been cut into portions (117), the system comprising:
a first conveyor (665);
at least one spray-removal robot (651);
a first receiving conveyor (667) and a second receiving conveyor (668), defining a space therebetween; and
a third receiving conveyor (662),
wherein the spray-removal robot (651) comprises at least one valve (661) with a nozzle (663), wherein the valve (661) is configured to eject fluid through the nozzle (663) to impact a food article (101) being moved on the first conveyor (665) toward the third receiving conveyor (662), such that at least one portion (117) of the food article (101) is moved by the impact through the space between the first receiving conveyor (667) and the second receiving conveyor (668), and such that at least one portion (117) of the food article (101) is moved by the motion of the first receiving conveyor (667) and the second receiving conveyor (668) to the third receiving conveyor (662).

2. The system for grading a food article (101) that has been cut into portions (117) of claim 1, wherein the spray-removal robot (651) comprises a plurality of valves (661) with nozzles (663, 666, 672, 674, 676).

3. The system for grading a food article (101) that has been cut into portions (117) of claim 2, wherein each of the plurality of valves (661) with nozzles (663, 666, 672, 674, 676) is independently controllable;
and/or
wherein the system comprises a plurality of the spray-removal robots (651).

4. The system for grading a food article (101) that has been cut into portions (17) of any of claims 1-3, wherein the first receiving conveyor (667) and the second receiving conveyor (668) are movable in a direction substantially perpendicular to the movement of the first conveyor (665), preferably wherein the movement of the first receiving conveyor (667) and the movement of the second receiving conveyor (668) are independently controllable.

5. The system for grading a food article (101) that has been cut into portions (117) of claim 4, wherein the movement of the first receiving conveyor (667) and the movement of the second receiving conveyor (668) are controlled by a computer that controls the spray-removal robot (651).

6. The system for grading a food article (101) that has been cut into portions (117) of any of claims 1-5, wherein the first receiving conveyor (667) and the second receiving conveyor (668) are rollers;
and/or
wherein the system further comprises a laser sensor (618).

7. A food processing apparatus (100; 200) comprising:
at least one conveyor (208, 214, 218, 226);
a cutting machine (216); and
the system for grading a food article (101) that has been cut into portions (117) of any of claims 1-6.

8. The food processing apparatus of claim 7, further comprising:
at least one element selected from the group consisting of
at least one imaging system (234, 235);
at least one realigning apparatus (802);
at least one x-ray machine (212, 217);
at least one manual quality check station (254);
at least one automated quality check station (244); and
at least one additional grading device (224).

9. The food processing apparatus (100, 200) of claim 8, wherein the additional grading device (224) is a needle grading device (600), a gripper grading device (601), a two degrees of freedom grading device (700), a three degrees of freedom grading device, or a four degrees of freedom grading device.

10. The food processing apparatus (100, 200) of any of claims 7-9, further comprising at least one computer programmed to operate the spray-removal robot (651) according to one or more inputs selected from the group consisting of an x-ray image, 3D image, a signal from a cutting robot (400), the movement of a grading conveyor (218), and the movement of any other conveyor(s) preceding the spray-removal robot (651).

11. A method for grading a food article (101) that has been cut into portions (117), the method comprising utilizing the system for grading a food article (101) of any of claims 1-6 to remove at least one of the portions (117) of the food article (101) through the space defined between the first receiving conveyor (667) and the second receiving conveyor (668), while transferring the remaining portions (117) of the food article (101) to the third receiving conveyor (662).

12. The method according to claim 11, wherein the action of the spray-removal robot (651) is controlled by a computer according to a determination of a location of the portion (117) to be removed, wherein the computer determines the location of the portion (117) to be removed from one or more inputs selected from the group consisting of an x-ray image, 3D image, a signal from a cutting robot (400), the movement of a grading conveyor (218), and the movement of any other conveyor(s) preceding the spray-removal robot (651).

13. The method according to claim 11 or claim 12, wherein each of the portions (117) has an alignment and orientation with respect to the surface of the first conveyor (665), and are positioned parallel to each other on the surface of the first conveyor (665),
wherein none of the portions (117) that are not removed and are positioned parallel to each other on the surface of the first conveyor (665) are substantially moved during the removal of the portion (117) through the space defined between the first receiving conveyor (667) and the second receiving conveyor (668), and
wherein the alignments and orientations of the portions (117) that are not removed and are positioned parallel to each other on the surface of the first conveyor (665) are substantially maintained after the removal of the portion (117) through the space defined between the first receiving conveyor (667) and the second receiving conveyor (668), and the transfer of the remaining portions (117) that are not removed to the third receiving conveyor (662).

14. The method according to claim 13, wherein the portion (117) that is removed is closely adjoined on opposing sides in an orientation substantially perpendicular to the parallel orientation of the plurality of portions (117) by other portions.

15. The method according to any of claims 11-14, further comprising selecting the portion (117) to be removed based on at least one criteria selected from the group consisting of size, weight, quality, type of portion, and type of comprising bone.

## Patentansprüche

1. System zum Sortieren eines Lebensmittelartikels (101), der in Portionen (117) geschnitten worden ist, wobei das System aufweist:
einen ersten Förderer (665);
mindestens einen Sprühentfernungsroboter (651);
einen ersten Aufnahmeförderer (667) und einen zweiten Aufnahmeförderer (668), die einen Raum dazwischen definieren; und
einen dritten Aufnahmeförderer (662),
wobei der Sprühentfernungsroboter (651) mindestens ein Ventil (661) mit einer Düse (663) aufweist, wobei das Ventil (661) dazu eingerichtet ist, ein Fluid durch die Düse (663) auszustoßen, um auf einen Lebensmittelartikel (101) aufzuprallen, der auf dem ersten Förderer (665) zum dritten Aufnahmeförderer (662) bewegt wird, so dass mindestens eine Portion (117) des Lebensmittelartikels (101) durch den Aufprall durch den Raum zwischen dem ersten Aufnahmeförderer (667) und dem zweiten Aufnahmeförderer (668) bewegt wird, und so dass mindestens eine Portion (117) des Lebensmittelartikels (101) durch die Bewegung des ersten Aufnahmeförderers (667) und des zweiten Aufnahmeförderers (668) zum dritten Aufnahmeförderer (662) bewegt wird.

2. System zum Sortieren eines Lebensmittelartikels (101), der in Portionen (117) geschnitten worden ist, nach Anspruch 1, wobei der Sprühentfernungsroboter (651) mehrere Ventile (661) mit Düsen (663, 666, 672, 674, 676) aufweist.

3. System zum Sortieren eines Lebensmittelartikels (101), der in Portionen (117) geschnitten worden ist, nach Anspruch 2, wobei jedes der mehreren Ventile (661) mit Düsen (663, 666, 672, 674, 676) unabhängig voneinander steuerbar ist;
und/oder
wobei das System mehrere Sprühentfernungsroboter (651) aufweist.

4. System zum Sortieren eines Lebensmittelartikels (101), der in Portionen (117) geschnitten worden ist, nach einem der Ansprüche 1 bis 3, wobei der erste Aufnahmeförderer (667) und der zweite Aufnahmeförderer (668) in einer im Wesentlichen zur Bewegung des ersten Förderers (665) senkrechten Richtung beweglich sind, wobei vorzugsweise die Bewegung des ersten Aufnahmeförderers (667) und die Bewegung des zweiten Aufnahmeförderers (668) unabhängig voneinander steuerbar sind.

5. System zum Sortieren eines Lebensmittelartikels (101), der in Portionen (117) geschnitten worden ist nach Anspruch 4, wobei die Bewegung des ersten Aufnahmeförderers (667) und die Bewegung des zweiten Aufnahmeförderers (668) von einem Computer, der den Sprühentfernungsroboter (651) steuert, gesteuert werden.

6. System zum Sortieren eines Lebensmittelartikels (101), der in Portionen (117) geschnitten worden ist, nach einem der Ansprüche 1 bis 5, wobei der erste Aufnahmeförderer (667) und der zweite Aufnahmeförderer (668) Rollen sind; und/oder
wobei das System ferner einen Lasersensor (618) aufweist.

7. Lebensmittelverarbeitungsvorrichtung (100; 200), die aufweist:
mindestens einen Förderer (208, 214, 218, 226);
eine Schneidemaschine (216); und
das System zum Sortieren eines Lebensmittelartikels (101), der in Portionen (117) geschnitten worden ist, nach einem der Ansprüche 1 bis 6.

8. Lebensmittelverarbeitungsvorrichtung nach Anspruch 7, die zusätzlich aufweist:
mindestens ein Element, ausgewählt aus der Gruppe bestehend aus:
mindestens einem bildgebenden System (234, 235);
mindestens einer Neuausrichtungsvorrichtung (802);
mindestens einem Röntgengerät (212, 217);
mindestens einer manuellen Qualitätskontrollstation (254);
mindestens einer automatischen Qualitätskontrollstation (244); und
mindestens einer zusätzlichen Sortiervorrichtung (224).

9. Lebensmittelverarbeitungsvorrichtung (100, 200) nach Anspruch 8, wobei die zusätzliche Sortiervorrichtung (224) eine Nadel-Sortiervorrichtung (600), eine Greifersortiervorrichtung (601), eine Sortiervorrichtung mit zwei Freiheitsgraden (700), eine Sortiervorrichtung mit drei Freiheitsgraden oder eine Sortiervorrichtung mit vier Freiheitsgraden ist.

10. Lebensmittelverarbeitungsvorrichtung (100, 200) nach einem der Ansprüche 7 bis 9, die zusätzlich mindestens einen Computer aufweist, der programmiert ist, den Sprühentfernungsroboter (651) entsprechend einem oder mehreren Eingangssignalen zu betreiben, die ausgewählt sind aus der Gruppe bestehend aus einem Röntgenbild, einem 3D-Bild, einem Signal von einem Schneidroboter (400), der Bewegung eines Sortierförderers (218) und der Bewegung eines oder mehrerer anderer Förderer, die dem Sprühentfernungsroboter (651) vorausgehen.

11. Verfahren zum Sortieren eines Lebensmittelartikels (101), der in Portionen (117) geschnitten worden ist, wobei das Verfahren das Verwenden des Systems zum Sortieren eines Lebensmittelartikels (101) nach einem der Ansprüche 1 bis 6 aufweist, um mindestens eine der Portionen (117) des Lebensmittelartikels (101) durch den zwischen dem ersten Aufnahmeförderer (667) und dem zweiten Aufnahmeförderer (668) definierten Raum zu entfernen, während die verbleibenden Portionen (117) des Lebensmittelartikels (101) zum dritten Aufnahmeförderer (662) transferiert werden.

12. Verfahren nach Anspruch 11, wobei die Aktion des Sprühentfernungsroboters (651) von einem Computer gemäß einer Bestimmung eines Ortes der zu entfernenden Portion (117) gesteuert wird, wobei der Computer den Ort der zu entfernenden Portion (117) aus einem oder mehreren Eingangssignalen bestimmt, die ausgewählt sind aus der Gruppe bestehend aus einem Röntgenbild, einem 3D-Bild, einem Signal von einem Schneidroboter (400), der Bewegung eines Sortierförderers (218) und der Bewegung eines oder mehrerer anderer Förderer, die dem Sprühentfernungsroboter (651) vorausgehen.

13. Verfahren nach Anspruch 11 oder Anspruch 12, wobei jede der Portionen (117) eine Ausrichtung und Orientierung in Bezug auf die Oberfläche des ersten Förderers (665) aufweist und parallel zueinander auf der Oberfläche des ersten Förderers (665) angeordnet ist,
wobei keine der Portionen (117), die nicht entfernt werden und parallel zueinander auf der Oberfläche des ersten Förderers (665) positioniert sind, während der Entfernung der Portion (117) durch den zwischen dem ersten Aufnahmeförderer (667) und dem zweiten Aufnahmeförderer (668) definierten Raum wesentlich bewegt wird, und
wobei die Ausrichtungen und Orientierungen der Portionen (117), die nicht entfernt werden und parallel zueinander auf der Oberfläche des ersten Förderers (665) positioniert sind, nach der Entfernung der Portion (117) durch den zwischen dem ersten Aufnahmeförderer (667) und dem zweiten Aufnahmeförderer (668) definierten Raum und dem Transfer der restlichen Portionen (117), die nicht entfernt werden, zum dritten Aufnahmeförderer (662) im Wesentlichen beibehalten werden.

14. Verfahren nach Anspruch 13, wobei die Portion (117), die entfernt wird, an gegenüberliegenden Seiten in einer Ausrichtung im Wesentlichen senkrecht zur parallelen Ausrichtung der Vielzahl von Portionen (117) durch andere Portionen eng anliegt.

15. Verfahren nach einem der Ansprüche 11 bis 14, zusätzlich aufweisend das Auswählen der zu entfernenden Portion (117), basierend auf mindestens einem Kriterium, das ausgewählt ist aus der Gruppe bestehend aus Größe, Gewicht, Qualität, Art der Portion und Art des enthaltenen Knochens.

## Revendications

1. Système pour trier un article alimentaire (101) qui a été coupé en portions (117), le système comprenant :
un premier transporteur (665) ;
au moins un robot de suppression de pulvérisation (651) ;
un premier transporteur de réception (667) et un deuxième transporteur de réception (668), définissant un espace entre eux ; et
un troisième transporteur de réception (662),
dans lequel le robot de suppression de pulvérisation (651) comprend au moins une valve (661) avec une buse (663), dans lequel la valve (661) est configurée pour éjecter du fluide par la buse (663) pour impacter un article alimentaire (101) qui est déplacé sur le premier transporteur (665) vers le troisième transporteur de réception (662), de sorte qu'au moins une portion (117) de l'article alimentaire (101) est déplacée par l'impact à travers l'espace entre le premier transporteur de réception (667) et le deuxième transporteur de réception (668), et de sorte qu'au moins une portion (117) de l'article alimentaire (101) est déplacée par le mouvement du premier transporteur de réception (667) et du deuxième transporteur de réception (668) jusqu'au troisième transporteur de réception (662).

2. Système pour trier un article alimentaire (101) qui a été coupé en portions (117) selon la revendication 1, dans lequel le robot de suppression de pulvérisation (651) comprend une pluralité de valves (661) avec des buses (663, 666, 672, 674, 676).

3. Système pour trier un article alimentaire (101) qui a été coupé en portions (117) selon la revendication 2, dans lequel chacune de la pluralité de valves (661) avec des buses (663, 666, 672, 674, 676) est indépendamment contrôlable ;
et/ou
dans lequel le système comprend une pluralité de robots de retrait de pulvérisation (651).

4. Système pour trier un article alimentaire (101) qui a été coupé en portions (117) selon l'une quelconque des revendications 1 à 3, dans lequel le premier transporteur de réception (667) et le deuxième transporteur de réception (668) sont mobiles dans une direction sensiblement perpendiculaire au mouvement du premier transporteur (665), de préférence dans lequel le mouvement du premier transporteur de réception (667) et le mouvement du deuxième transporteur de réception (668) sont indépendamment contrôlables.

5. Système pour trier un article alimentaire (101) qui a été coupé en portions (117) selon la revendication 4, dans lequel le mouvement du premier transporteur de réception (667) et le mouvement du deuxième transporteur de réception (668) sont contrôlés par un ordinateur qui contrôle le robot de suppression de pulvérisation (651).

6. Système pour trier un article alimentaire (101) qui a été coupé en portions (117) selon l'une quelconque des revendications 1 à 5, dans lequel le premier transporteur de réception (667) et le deuxième transporteur de réception (668) sont des rouleaux ;
et/ou
dans lequel le système comprend en outre un capteur laser (618).

7. Appareil de traitement alimentaire (100 ; 200) comprenant :
au moins un transporteur (208, 214, 218, 226) ;
une machine de coupe (216) ; et
le système pour trier un article alimentaire (101) qui a été coupé en portions (117) selon l'une quelconque des revendications 1 à 6.

8. Appareil de traitement alimentaire selon la revendication 7, comprenant en outre :
au moins un élément sélectionné dans le groupe comprenant :
au moins un système d'imagerie (234, 235) ;
au moins un appareil de réalignement (802) ;
au moins une machine à rayons X (212, 217) ;
au moins une station de contrôle qualité manuelle (254) ;
au moins une station de contrôle qualité automatique (244) ; et
au moins un dispositif de tri (224) supplémentaire.

9. Appareil de traitement alimentaire (100, 200) selon la revendication 8, dans lequel le dispositif de tri (224) supplémentaire est un dispositif de tri à aiguille (600), un dispositif de tri à pince (601), un dispositif de tri à deux degrés de liberté (700), un dispositif de tri à trois degrés de liberté ou un dispositif de tri à quatre degrés de liberté.

10. Appareil de traitement alimentaire (100, 200) selon l'une quelconque des revendications 7 à 9, comprenant en outre au moins un ordinateur programmé pour actionner le robot de suppression de pulvérisation (651) selon une ou plusieurs entrées sélectionnées dans le groupe comprenant une image radiologique, une image tridimensionnelle, un signal provenant d'un robot de coupe (400), le mouvement d'un transporteur de tri (218), et le mouvement de l'un quelconque des autres transporteurs précédant le robot de suppression de pulvérisation (651).

11. Procédé pour trier un article alimentaire (101) qui a été coupé en portions (117), le procédé comprenant l'utilisation du système pour trier un article alimentaire (101) selon l'une quelconque des revendications 1 à 6 afin de retirer au moins l'une des portions (117) de l'article alimentaire (101) par l'espace défini entre le premier transporteur de réception (667) et le deuxième transporteur de réception (668), tout en transférant les parties résiduelles (117) de l'article alimentaire (101) au troisième transporteur de réception (662).

12. Procédé selon la revendication 11, dans lequel l'action du robot de suppression de pulvérisation (651) est contrôlée par un ordinateur selon une détermination d'un emplacement de la portion (117) à retirer, dans lequel l'ordinateur détermine l'emplacement de la portion (117) à retirer d'après une ou plusieurs entrées sélectionnées dans le groupe comprenant une image radiographique, une image tridimensionnelle, un signal provenant d'un robot de coupe (400), le mouvement d'un transporteur de tri (218) et le mouvement de l'un quelconque des autres transporteurs précédant le robot de suppression de pulvérisation (651).

13. Procédé selon la revendication 11 ou la revendication 12, dans lequel chacune des portions (117) a un alignement et une orientation par rapport à la surface du premier transporteur (665) et est positionnée parallèlement à l'autre sur la surface du premier transporteur (665),
dans lequel aucune des portions (117) qui ne sont pas retirées et sont positionnées parallèlement entre elles sur la surface du premier transporteur (665) n'est sensiblement déplacée pendant le retrait de la portion (117) par l'espace défini entre le premier transporteur de réception (667) et le deuxième transporteur de réception (668), et
dans lequel les alignements et les orientations des portions (117) qui ne sont pas retirées et sont positionnées parallèlement entre elles sur la surface du premier transporteur (665) sont sensiblement maintenus après le retrait de la portion (117) à travers l'espace défini entre le premier transporteur de réception (667) et le deuxième transporteur de réception (668), et le transfert des portions résiduelles (117) qui ne sont pas retirées, au troisième transporteur de réception (662).

14. Procédé selon la revendication 13, dans lequel la portion (117) qui est retirée, est étroitement attenante sur les côtés opposés dans une orientation sensiblement perpendiculaire à l'orientation parallèle de la pluralité de portions (117) grâce à d'autres portions.

15. Procédé selon l'une quelconque des revendications 11 à 14, comprenant en outre la sélection de la portion (117) à retirer sur la base d'au moins un critère sélectionné dans le groupe comprenant la taille, le poids, la qualité, le type de portion et le type d'os de constitution.
